# EUROPEAN PATENT APPLICATION

(11) **EP 4 818 184 A2**
(43) Date of publication of application: **07.10.2026**
(21) Application number: 26169387.3
(22) Date of filing: 31.03.2026
(51) Int. Cl.: C07H 1/00, C07H 15/203, A61P 35/00, C07K 9/00, C07K 1/00

(54) **METHOD FOR PREPARING A URONYL COMPOUND**

(30) Priority: 04.04.2025 FI 20255311
(71) Applicant: Glykos Finland Oy, 00790 Helsinki (FI)
(72) Inventor: HELIN, Jari, 05200 Rajamäki (FI); SATOMAA, Tero, 00700 Helsinki (FI); SAARINEN, Juhani, 00370 Helsinki (FI)
(74) Representative: Papula Oy

(57) **Abstract**

A method for preparing a uronyl compound is disclosed.

## Description

### TECHNICAL FIELD

The present disclosure relates to a method for preparing a uronyl compound and to a compound.

### BACKGROUND

Glucuronyl-monomethylauristatin E (MMAU) is a promising payload molecule that may be incorporated into antibody-drug conjugates and delivered to target cells. The glucuronyl moiety renders the MMAU more hydrophilic. MMAU and related compounds may be synthesized using methods in which the glucuronyl moiety is added into MMAE. Such methods, however, are not particularly efficient.

Thus, the present disclosure provides novel methods for preparing MMAU and similar compounds.

### SUMMARY

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter.

A method for preparing a uronyl compound is disclosed. The method may comprise providing a compound represented by Formula I 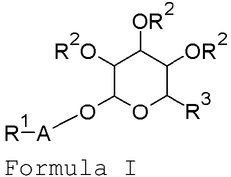
wherein R¹ is either H or a protecting group;
A is selected from A¹, A², A³, A⁴, and A⁵ 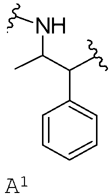 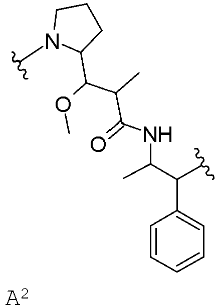 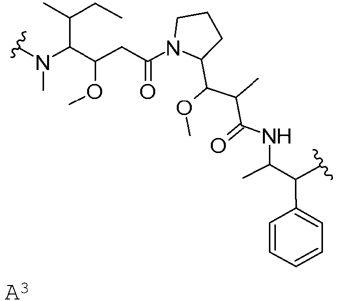 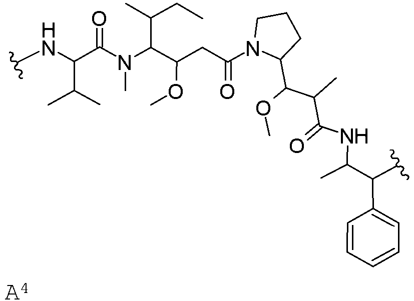 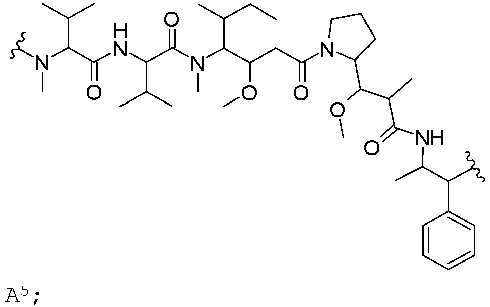
R³ is CH₂OH or CH₂OR²; and
each R² is independently selected from either H or a protecting group;
when R³ is CH₂OR², wherein R² is a protecting group, optionally removing the protecting group from R³; and
oxidizing the compound represented by Formula I in the presence of an oxidizing agent, such that R³ in the compound represented by Formula I is oxidized into COOH.

### BRIEF DESCRIPTION OF THE DRAWINGS

The accompanying drawings, which are included to provide a further understanding of the described embodiments and constitute a part of this specification, illustrate various advantageous features and examples of their combinations. In the drawings:
**Figure 1** illustrates RP-HPLC (reversed-phase high-performance liquid chromatography) of synthesized MMAU indicating 99.83% purity;
**Figure 2** shows an ESI-MS (electrospray ionization mass spectrometry) spectrum of synthesized MMAU. [M+H]⁺ observed at m/z 894.7 (calculated m/z 894.54) and [M+2H]²⁺ observed at m/z 447.7 (calculated m/z 447.78). y-axis shows the signal intensity;
**Figure 3** shows a ¹³C-edited HSQC (heteronuclear single quantum coherence) NMR (nuclear magnetic resonance) spectroscopic analysis of MMAU showing signals arising from the β-glucuronic acid; and
**Figure 4** illustrates ¹³C-edited HSQC NMR spectroscopic analysis of MMAE. The result is essentially similar to the full assignment of MMAE as described in Johansson et al. 2017 Sci Rep 7:15920, including the cis/trans-equilibrium at dolaisoleuine-dolaproine amide bond giving rise to distinct sets of peaks for both conformers, which applies also for MMAU (Figure 3).

### DETAILED DESCRIPTION

A method for preparing a uronyl compound is disclosed.

The method may comprise providing a compound represented by Formula I 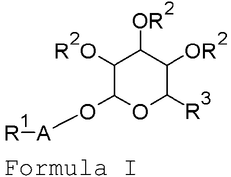
wherein R¹ is either H or a protecting group;
A is selected from A¹, A², A³, A⁴, and A⁵ 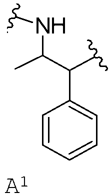 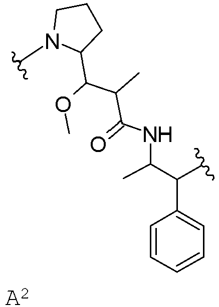 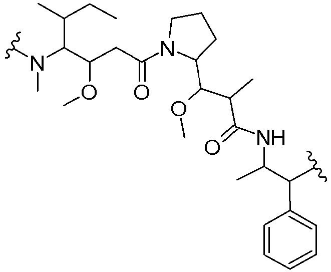 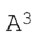 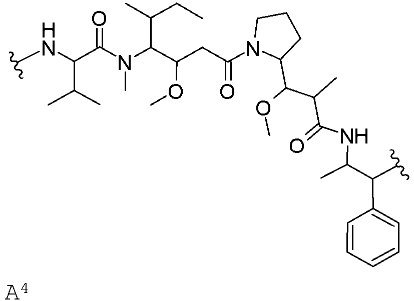 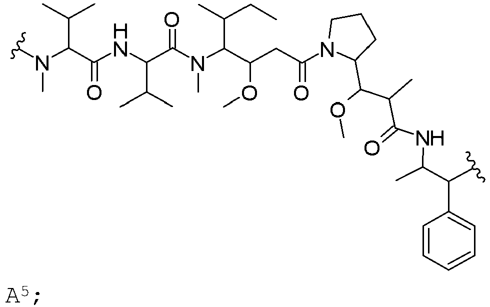
R³ is CH₂OR², CH₂OH, or OH; and
each R² is independently selected from either H or a protecting group;
when R³ is CH₂OR², wherein R² is a protecting group, optionally removing the protecting group from R³; and
oxidizing the compound represented by Formula I in the presence of an oxidizing agent, such that R³ in the compound represented by Formula I is oxidized into COOH, i.e. into a carboxyl group. The uronyl compound may thus be obtained. However, the uronyl compound obtained by the oxidizing may be reacted further, for example so as to prepare a uronyl-monomethylauristatin compound therefrom.

A method for preparing a uronyl compound is also disclosed, wherein the method comprises
providing a compound represented by Formula I 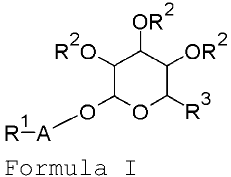
wherein R¹ is either H or a protecting group;
A is selected from A¹, A², A³, A⁴, and A⁵ 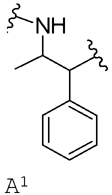 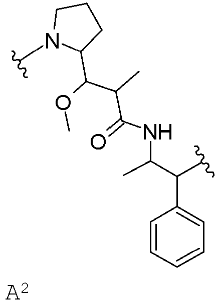 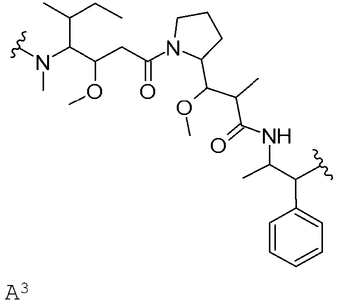 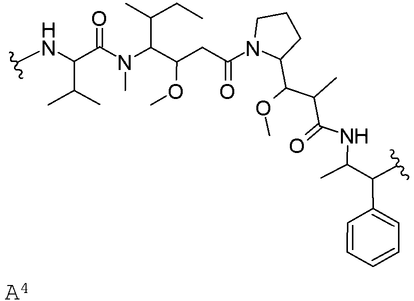 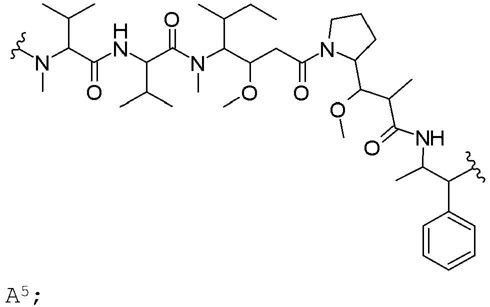
R³ is CH₂OH or CH₂OR²; and
each R² is independently selected from either H or a protecting group;
when R³ is CH₂OR², wherein R² is a protecting group, optionally removing the protecting group from R³; and
oxidizing the compound represented by Formula I in the presence of an oxidizing agent, a catalyst, water, and a bromide, such that R³ in the compound represented by Formula I is oxidized into COOH, wherein the oxidizing agent is sodium hypochlorite and the catalyst is 2,2,6,6-tetramethylpiperidine 1-oxyl (TEMPO).The method may be a method for preparing a uronyl-monomethylauristatin compound.

In the context of this specification, the term "protecting group", unless otherwise stated or implied by context, may be understood as referring to a moiety that prevents or substantially reduces the ability of the atom or functional group to which it is linked from participating in unwanted reactions. Protecting groups for heteroatoms such as oxygen, sulfur and nitrogen are sometime used to minimize or avoid their unwanted reactions with electrophilic compounds. Other times the protecting group is used to reduce or eliminate the nucleophilicity and/or basicity of the unprotected heteroatom. Non-limiting examples of protected oxygen are given by -OR^{PR}, wherein R^{PR} is a protecting group for hydroxyl, wherein hydroxyl is typically protected as an ester (e.g., acetate, propionate or benzoate). Other protecting groups for hydroxyl avoid its interference with the nucleophilicity of organometallic reagents or other highly basic reagents, for which purpose hydroxyl is typically protected as an ether, including without limitation alkyl or heterocyclyl ethers, (e.g., methyl or tetrahydropyranyl ethers), alkoxymethyl ethers (e.g., methoxymethyl or ethoxymethyl ethers), optionally substituted aryl ethers, and silyl ethers (e.g., trimethylsilyl (TMS), triethylsilyl (TES), tert-butyldiphenylsilyl (TBDPS), tert-butyldimethylsilyl (TBS/TBDMS), triisopropylsilyl (TIPS) and [2-(trimethylsilyl)ethoxy]-methylsilyl (SEM)). Nitrogen protecting groups include those for primary or secondary amines as in -NHR^{PR} or -N(R^{PR})², wherein least one of R^{PR} is a nitrogen atom protecting group or both R^{PR} together define a nitrogen atom protecting group.

A protecting group is a suitable for protecting when it is capable of preventing or substantially avoiding unwanted side-reactions and/or premature loss of the protecting group under reaction conditions required to effect desired chemical transformation(s) elsewhere in the molecule and during purification of the newly formed molecule when desired, and can be removed under conditions that do not adversely affect the structure or stereochemical integrity of that newly formed molecule. In some embodiments, suitable protecting groups may be those previously described for protecting functional groups. In some embodiments, a suitable protecting group is a protecting group used in peptide coupling reactions. For example, a suitable protecting group for the basic nitrogen atom of an acyclic or cyclic basic group is an acid-labile carbamate protecting group such as t-butyloxycarbonyl (Boc).

The protecting group which may be R¹ may be Boc, i.e. a tert-butyloxycarbonyl protecting group, PNZ, i.e. a para-nitro-benzyloxy protecting group, or Fmoc, i.e. a fluorenylmethoxycarbonyl protecting group. In other words, R¹ may be selected from Boc, PNZ, and Fmoc. The Boc group can be added to amines under aqueous conditions using di-tert-butyl dicarbonate in the presence of a base, such as sodium hydroxide. The PNZ group can be added to amines under aqueous conditions using *para*-nitrolbenzyl chloroformate in the presence of a base, such as sodium hydroxide. The Fmoc group can be added to amines under aqueous conditions using Fmoc-N-hydroxysuccinimide ester (Fmoc-NHS) in the presence of a base, such as sodium bicarbonate.

The protecting group which may be R² may be Bz or 2, 5-difluoro-Bz, i.e. a benzoyl group or a 2,5-difluorobenzoyl group. In some embodiments, each R² may be independently selected from H, Bz, and 2,5-difluoro-Bz. In some embodiments, each R² may be independently selected from H and Bz or from H and 2,5-difluoro-Bz. In some embodiments, each R² may be H. In some embodiments, each R² may be Bz. In some embodiments, each R² may be 2,5-difluoro-Bz. In some embodiments, at least one R² is H. Other possible protecting groups which may be R² may be alkyl or heterocyclyl ethers, (e.g., methyl or tetrahydropyranyl ethers), alkoxymethyl ethers (e.g., methoxymethyl or ethoxymethyl ethers), optionally substituted aryl ethers, and silyl ethers (e.g., trimethylsilyl (TMS), triethylsilyl (TES), tert-butyldiphenylsilyl (TBDPS), tert-butyldimethylsilyl (TBS/TBDMS), triisopropylsilyl (TIPS) and [2-(trimethylsilyl)ethoxy]-methylsilyl (SEM)).

In some embodiments, R¹ is Boc or Fmoc. In some embodiments, R¹ is Boc. In some embodiments, R¹ is Fmoc.

In some embodiments, R³ is CH²OH or CH²OR².

In some embodiments, each R² is Bz or H. In some embodiments, each R² is Bz. In some embodiments, each R² is H.

In an embodiment, R³ is CH²OH or CH²OR²; R¹ is Boc or Fmoc; and each R² is Bz or H.

In an embodiment, R³ is CH²OH or CH²OR²; R¹ is Boc; and each R² is Bz or H.

In an embodiment, R³ is CH²OH or CH²OR²; R¹ is Fmoc; and each R² is Bz or H.

In an embodiment, R³ is CH²OH or CH²OR²; R¹ is Boc or Fmoc; and each R² is Bz.

In an embodiment, R³ is CH²OH or CH²OR²; R¹ is Boc or Fmoc; and each R² is H.

In an embodiment, R³ is CH²OR²; R¹ is Boc or Fmoc; and each R² is Bz or H.

In an embodiment, R³ is CH²OR²; R¹ is Boc; and each R² is Bz or H.

In an embodiment, R³ is CH²OR²; R¹ is Fmoc; and each R² is Bz or H.

In an embodiment, R³ is CH²OR²; R¹ is Boc or Fmoc; and each R² is Bz.

In an embodiment, R³ is CH²OR²; R¹ is Boc or Fmoc; and each R² is H.

In an embodiment, R³ is CH²OH; R¹ is Boc or Fmoc; and each R² is Bz or H.

In an embodiment, R³ is CH²OH; R¹ is Boc; and each R² is Bz or H.

In an embodiment, R³ is CH²OH; R¹ is Fmoc; and each R² is Bz or H.

In an embodiment, R³ is CH²OH; R¹ is Boc or Fmoc; and each R² is Bz.

In an embodiment, R³ is CH²OH; R¹ is Boc or Fmoc; and each R² is H.

In an embodiment, R³ is CH²OH or CH²OR²; R¹ is Boc or Fmoc; each R² is Bz or H; and A is A¹ or A².

In an embodiment, R³ is CH²OH or CH²OR²; R¹ is Boc; each R² is Bz or H; and A is A¹ or A².

In an embodiment, R³ is CH²OH or CH²OR²; R¹ is Fmoc; each R² is Bz or H; and A is A¹ or A².

In an embodiment, R³ is CH²OH or CH²OR²; R¹ is Boc or Fmoc; each R² is Bz; and A is A¹ or A².

In an embodiment, R³ is CH²OH or CH²OR²; R¹ is Boc or Fmoc; each R² is H; and A is A¹ or A².

In an embodiment, R³ is CH²OR²; R¹ is Boc or Fmoc; each R² is Bz or H; and A is A¹ or A².

In an embodiment, R³ is CH²OR²; R¹ is Boc; each R² is Bz or H; and A is A¹ or A².

In an embodiment, R³ is CH²OR²; R¹ is Fmoc; each R² is Bz or H; and A is A¹ or A².

In an embodiment, R³ is CH²OR²; R¹ is Boc or Fmoc; each R² is Bz; and A is A¹ or A².

In an embodiment, R³ is CH²OR²; R¹ is Boc or Fmoc; each R² is H; and A is A¹ or A².

In an embodiment, R³ is CH²OH; R¹ is Boc or Fmoc; each R² is Bz or H; and A is A¹ or A².

In an embodiment, R³ is CH²OH; R¹ is Boc; each R² is Bz or H; and A is A¹ or A².

In an embodiment, R³ is CH²OH; R¹ is Fmoc; each R² is Bz or H; and A is A¹ or A².

In an embodiment, R³ is CH²OH; R¹ is Boc or Fmoc; each R² is Bz; and A is A¹ or A².

In an embodiment, R³ is CH²OH; R¹ is Boc or Fmoc; each R² is H; and A is A¹ or A².

The compound represented by Formula I may be represented by e.g. any one of the formulas below and/or a compound selected from the following: 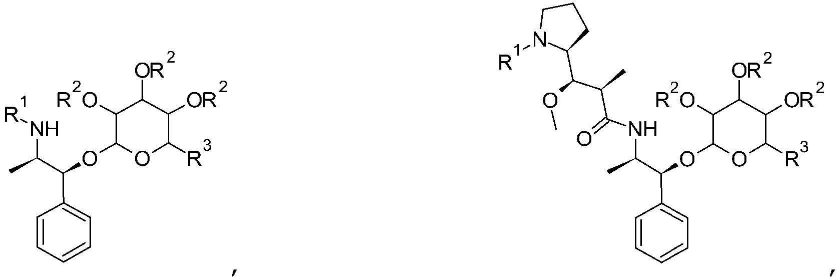 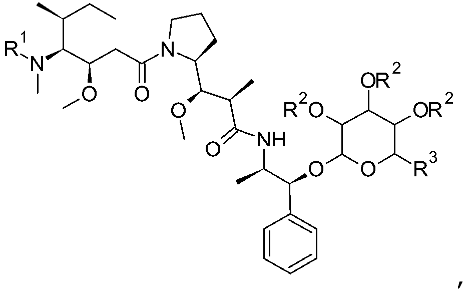 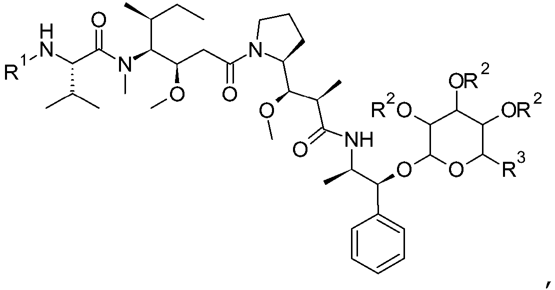 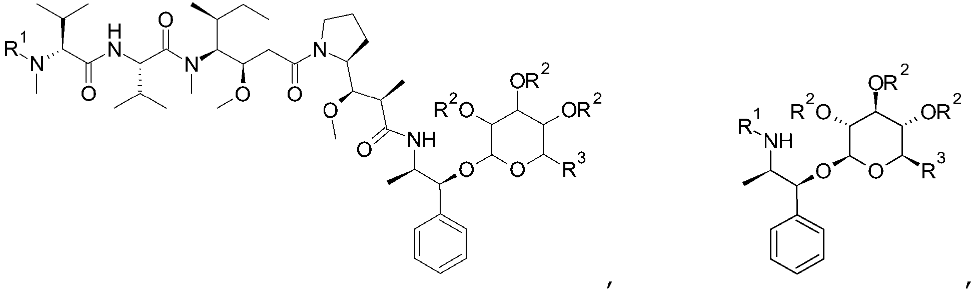 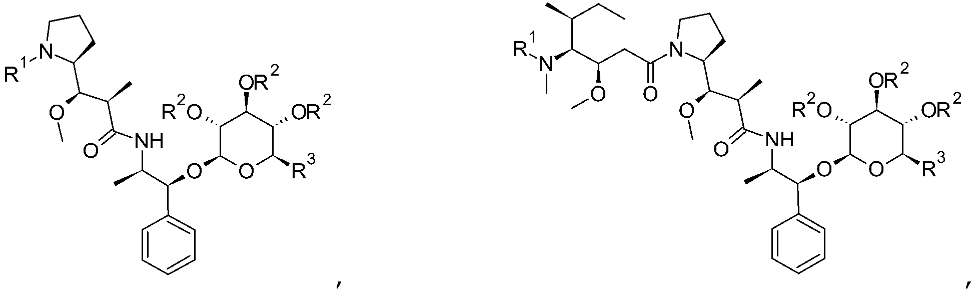 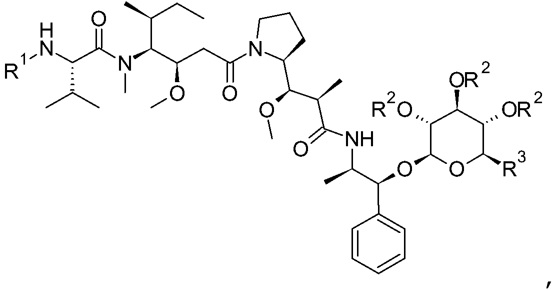 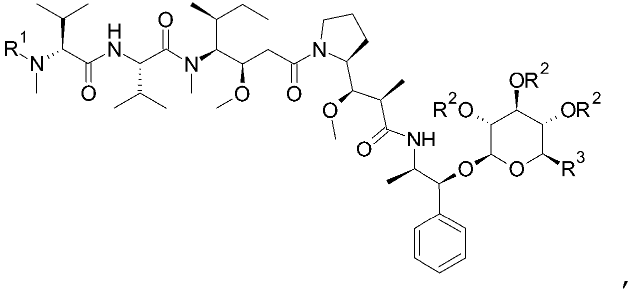 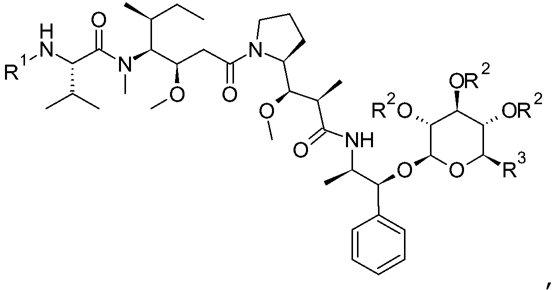 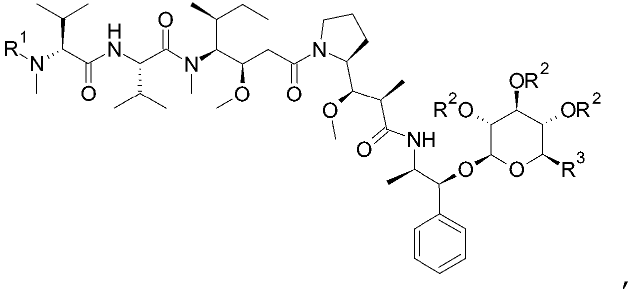 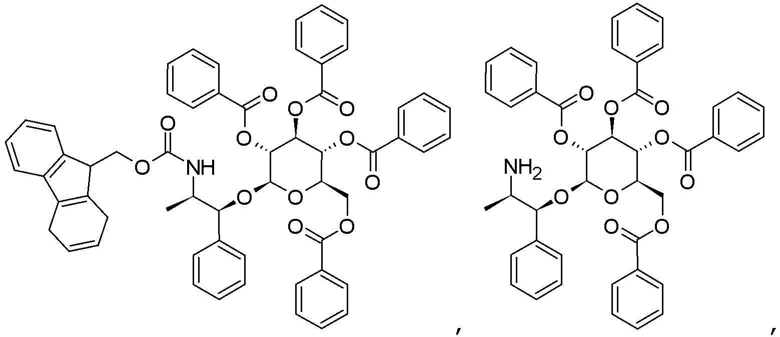 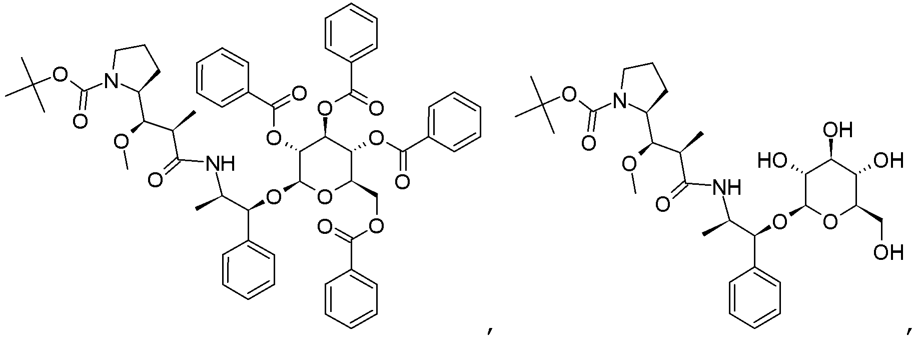 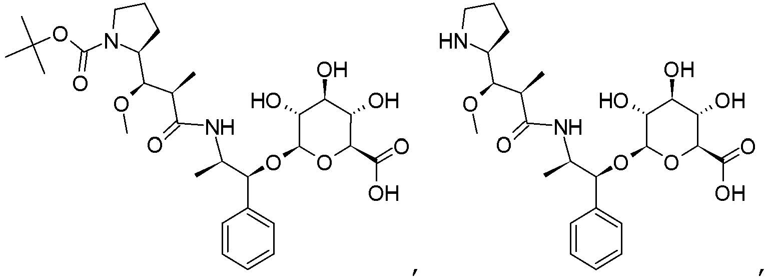 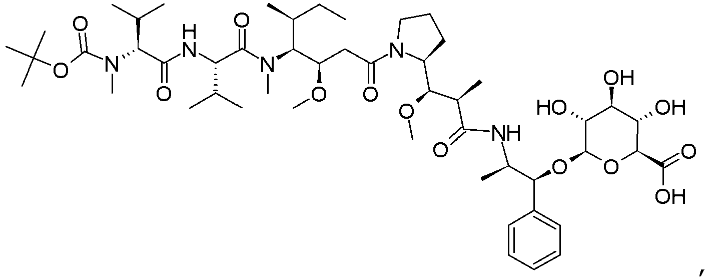 or 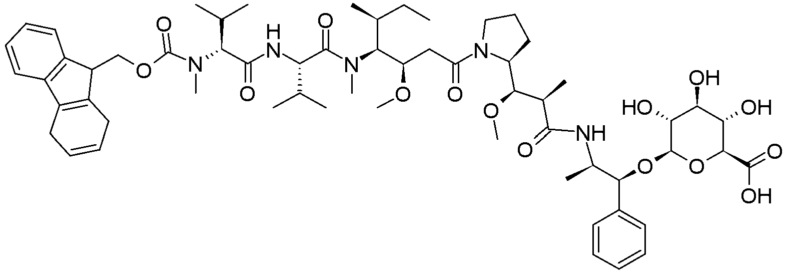 wherein R¹, R², and R³ are according to one or more embodiments disclosed in this specification (e.g. as defined above).

The compound represented by Formula I may be a compound selected from the following:

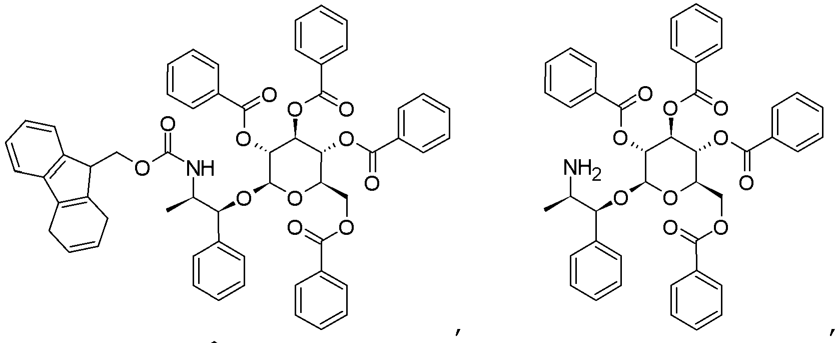

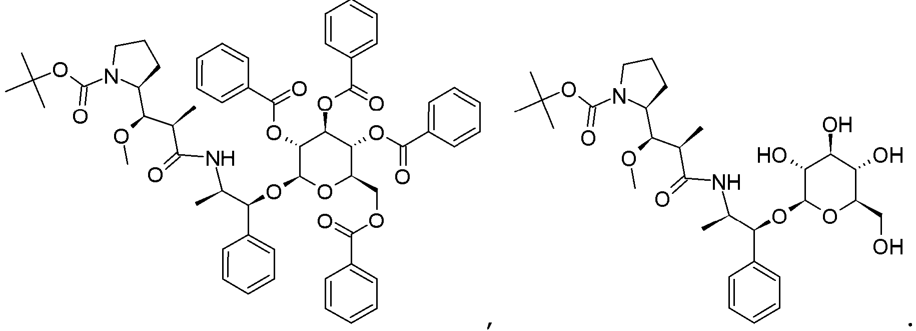

When R³ is CH₂OR², the R² in R³ being CH₂OR² (i.e. the protecting group attached to the primary hydroxyl) may be a protecting group. It may be any protecting group described in this specification. Each R², including the R² in R³, may be independently selected. In some embodiments, the R² in R³ may be the same protecting group as the other instances of R² (i.e. the R² groups attached to the secondary hydroxyls). In some embodiments, the R² in R³ may be a protecting group different from the protecting group(s) represented by other instances of R².

When R³ is CH₂OR², wherein R² is a protecting group, the method may further comprise removing the protecting group at least from R³.

When at least one of R¹ or R² is a protecting group, the method may comprise removing one or more of the at least one protecting group, or all protecting groups, prior to the oxidizing.

When at least one R² is a protecting group, the method may comprise removing one or more of the at least one protecting group(s), or all protecting groups, prior to the oxidizing.

When R³ is CH₂OR², wherein R² is a protecting group, the method may further comprise removing the protecting group from R³ or at least from R³. Thus, after the removal of the protecting group from R³, R³ may be understood as being CH²OH in the compound represented by Formula I. The CH²OH group may subsequently be oxidized into COOH.

In other words, when providing the compound represented by Formula I, R³ in the compound may be CH₂OR², wherein R² is a protecting group; then the method may further comprise removing the protecting group R² from R³ to thereby obtain a compound represented by Formula I, wherein R³ is CH²OH; and oxidizing the compound represented by Formula I according to one or more embodiments described in this specification.

In such an embodiment, the method may comprise removing one or more protecting groups from the compound represented by Formula I provided (including removing the protecting group R² from R³). For example, when each R² is a protecting group, the method may comprise removing each protecting group that is R² (i.e. all protecting groups represented by R²). The chemistry required may depend on the specific protecting group(s) to be removed. After the removal of all of the protecting groups represented by R², each instance of R² may be understood as being H.

The compound represented by Formula I may be oxidized in the presence of an oxidizing agent and optionally a catalyst.

The oxidizing agent may be selected from the group of hydrogen peroxide (H₂O₂), sodium hypochlorite (NaClO), calcium hypochlorite (Ca(ClO)₂), ozone (O₃), nitric acid, a permanganate compound (e.g. potassium permanganate), a halogen (e.g. Cl₂), a metal-catalyzed oxidation agent, gold nanoparticles, nanoparticles which mimic peroxidase activity-mimicking nanoparticles, and a glucose-oxidizing compound (e.g. glucose oxidase).

The oxidizing agent may be selected from sodium hypochlorite (NaClO), calcium hypochlorite (Ca(ClO)₂), and any combination or mixture thereof.

The oxidizing agent may comprise or be sodium hypochlorite (NaClO) in combination with sodium chlorite (NaClO₂).

The oxidizing agent may further comprise sodium chlorate (NaClO₃ ).

The oxidizing agent may be provided e.g. in the form of bleach, such as household bleach. Such a bleach may comprise sodium hypochlorite, optionally also sodium hypochlorite and/or sodium chlorate.

The oxidation may also occur via electrochemical oxidation in the presence or absence of a chemical catalyst, via gold or other nanoparticles which mimic peroxidase activity, via enzymatic oxidation or by an enzyme such as glucose oxidase, or via any other physicochemical means or condition, by which glucose could be forced to undergo electron donation. In fact, the oxidizing conditions of the present disclosure are intended to include any condition capable of oxidizing glucose.

In embodiments in which at least one R² is H, or in which each R² is H, it may be possible to perform the oxidizing such that only the hydroxyl in R³ (i.e. the primary hydroxyl) is oxidized into COOH, for example using a suitable catalyst.

The catalyst may be selected from the group consisting of 2,2,6,6-tetramethylpiperidine 1-oxyl (TEMPO), a derivative thereof, glucose oxidase, and a glucose-oxidizing enzyme.

The compound represented by Formula I may be oxidized in the presence of further components, such as a reaction accelerator. The reaction accelerator may also be referred to as an activator. Examples of suitable reaction accelerators may include e.g. a bromide, such as a bromide salt (e.g. NaBr, KBr or any mixture or combination thereof), chlorine dioxide and/or a tertiary amine. The term "bromide" may be understood as referring e.g. to a bromide salt, such as a bromide salt of an alkali metal, or a bromide compound capable of releasing Br⁻ ions in water (i.e. in an aqueous solution). The bromide may comprise or be e.g. NaBr, KBr or any mixture or combination thereof.

The catalyst may be selected from the group consisting of 2,2,6,6-tetramethylpiperidine 1-oxyl (TEMPO), and a derivative thereof. Examples of TEMPO derivatives may include 4-hydroxy-TEMPO, TEMPO methacrylate, 4-oxo-TEMPO, 4-amino-TEMPO, 4-acetamido-TEMPO, 4-carboxy-TEMPO, 4-hydroxy-TEMPO benzoate, 4-(2-iodoa-cetamido)-TEMPO, 4-maleimido-TEMPO, 4-isothiocyanato-TEMPO, 4-(2-bromoacetamido)-TEMPO, 4-methoxy-TEMPO, 4-cyano-TEMPO, 4-amino-4-carboxy-TEMPO, 4-phosphonooxy-TEMPO hydrate, and 2,2,6,6-tetramethyl-4-(methylsulfonyloxy)-1-piperidinooxy.

TEMPO and its derivatives are well suited as catalysts for the oxidation reaction. They allow for selectively oxidizing the primary hydroxyl group (i.e. R₃, which is CH₂OH) of the compound represented by Formula I to a carboxylic group.

The TEMPO or derivative thereof may be a free compound (radical) or linked to beads, a resin, or a polymer.

The catalyst may be selected from the group consisting of 2,2,6,6-tetramethylpiperidine 1-oxyl (TEMPO), 4-hydroxy-TEMPO, TEMPO methacrylate, 4-oxo-TEMPO, 4-amino-TEMPO, 4-acetamido-TEMPO, 4-carboxy-TEMPO, 4-hydroxy-TEMPO benzoate, 4-(2-iodoa-cetamido)-TEMPO, 4-maleimido-TEMPO, 4-isothiocyanato-TEMPO, 4-(2-bromoacetamido)-TEMPO, 4-methoxy-TEMPO, 4-cyano-TEMPO, 4-amino-4-carboxy-TEMPO, 4-phosphonooxy-TEMPO hydrate, and 2,2,6,6-tetramethyl-4-(methylsulfonyloxy)-1-piperidinooxy.

The catalyst may be 2,2,6,6-tetramethylpiperidine 1-oxyl (TEMPO) .

The oxidizing agent may selected from the group consisting of sodium hypochlorite and calcium hypochlorite, and the catalyst may be selected from the group consisting of 2,2,6,6-tetramethylpiperidine 1-oxyl (TEMPO), 4-hydroxy-TEMPO, TEMPO methacrylate, 4-oxo-TEMPO, 4-amino-TEMPO, 4-acetamido-TEMPO, 4-carboxy-TEMPO, 4-hydroxy-TEMPO benzoate, 4-(2-iodoacetamido)-TEMPO, 4-maleimido-TEMPO, 4-isothiocyanato-TEMPO, 4-(2-bromoacetamido)-TEMPO, 4-methoxy-TEMPO, 4-cyano-TEMPO, 4-amino-4-carboxy-TEMPO, 4-phosphonooxy-TEMPO hydrate, and 2,2,6,6-tetramethyl-4-(methyl-sulfonyloxy)-1-piperidinooxy.

The catalyst may be 2,2,6,6-tetramethylpiperidine 1-oxyl (TEMPO) or a derivative thereof, the oxidizing agent may be NaClO, and the compound represented by Formula I may be oxidized in the presence of the oxidizing agent, the catalyst, NaBr, and water.

The catalyst may be 2,2,6,6-tetramethylpiperidine 1-oxyl (TEMPO), the oxidizing agent may be NaClO, and the compound represented by Formula I may be oxidized in the presence of the oxidizing agent, the catalyst, NaBr, and water. In other words, the compound represented by Formula I may be oxidized in the presence of NaClO, TEMPO, NaBr, and water. Other compounds or components may also be present, if desired.

The catalyst may be TEMPO derivative, the oxidizing agent may be NaClO, and the compound represented by Formula I may be oxidized in the presence of the catalyst, oxidizing agent, NaBr or KBr, and water.

The catalyst may be a TEMPO derivative selected from 4-hydroxy-TEMPO, 4-oxo-TEMPO, 4-acetamido-TEMPO, or 4-carboxy-TEMPO, the oxidizing agent may be NaClO, and the compound represented by Formula I may be oxidized in the presence of the catalyst, oxidizing agent, NaBr or KBr, and water.

The catalyst may be TEMPO, the oxidizing agent may be calcium hypochlorite (Ca(ClO)₂), and the compound represented by Formula I may be oxidized in the presence of the catalyst, oxidizing agent, NaBr or KBr, and water.

The catalyst may be 4-hydroxy-TEMPO, the oxidizing agent may be NaClO or Ca(ClO)₂, and the compound represented by Formula I may be oxidized in the presence of the catalyst, oxidizing agent, NaBr or KBr, and water.

The catalyst may be TEMPO or a TEMPO derivative, the oxidizing agent may comprise sodium hypochlorite in combination with sodium chlorite (NaClO₂), and the compound represented by Formula I may be oxidized in the presence of the catalyst, oxidizing agent, NaBr or KBr, and water.

Hypochlorite (such as NaClO) may function as the main oxidant during the oxidizing using TEMPO or a derivative thereof as a catalyst. Other components or additives may also be included, such as a reaction accelerator.

The catalyst may be 2,2,6,6-tetramethylpiperidine 1-oxyl (TEMPO) or a derivative thereof, and the compound represented by Formula I may be oxidized in the presence of the catalyst, KBr or NaBr, oxidizing agent, and water.

The catalyst may be 2,2,6,6-tetramethylpiperidine 1-oxyl (TEMPO) or a derivative thereof, and the compound represented by Formula I may be oxidized in the presence of the catalyst, KBr, NaClO, and water.

The catalyst may be 2,2,6,6-tetramethylpiperidine 1-oxyl (TEMPO), and the compound represented by Formula I may be oxidized in the presence of the catalyst, KBr, NaClO, and water.

The catalyst may be 2,2,6,6-tetramethylpiperidine 1-oxyl (TEMPO), and the compound represented by Formula I may be oxidized in the presence of the catalyst, NaBr, NaClO, and water.

The catalyst may be TEMPO derivative, and the compound represented by Formula I may be oxidized in the presence of the catalyst, KBr or NaBr, oxidizing agent, and water.

The catalyst may be TEMPO derivative, and the compound represented by Formula I may be oxidized in the presence of the catalyst, KBr or NaBr, NaClO, and water.

The method may comprise providing an aqueous solution comprising the 2,2,6,6-tetramethylpiperidine 1-oxyl (TEMPO) or the derivative thereof, KBr, and water; mixing the compound represented by Formula I with the aqueous solution to obtain a mixture; adjusting the pH of the mixture; adding NaClO to the mixture, to thereby oxidize the compound represented by Formula I; optionally adjusting the pH of the mixture after the addition of the NaClO so as to keep the pH of the mixture essentially unchanged; and isolating the oxidized compound so that the oxidation degree of the compound is at least 80 %, or in the range of 90 - 100 %, 95 - 100 %, 98 - 100 %, 99 - 100 %, or about 100 %. The oxidation degree may be understood as referring the percentage (proportion) of the oxidized compound (i.e. in which R³ is COOH) based on the total combined amount (e.g. in mol) of the oxidized compound and the compound represented by Formula I that has not been oxidized (i.e. in which R³ is CH₂OH).

The method may comprise providing an aqueous solution comprising the 2,2,6,6-tetramethylpiperidine 1-oxyl (TEMPO), KBr, and water; mixing the compound represented by Formula I with the aqueous solution to obtain a mixture; adjusting the pH of the mixture; adding NaClO to the mixture, to thereby oxidize the compound represented by Formula I; adjusting the pH of the mixture after the addition of the NaClO so as to keep the pH of the mixture essentially unchanged; and isolating the oxidized compound so that the oxidation degree of the compound is at least 80 %.

The method may comprise providing an aqueous solution comprising the 2,2,6,6-tetramethylpiperidine 1-oxyl (TEMPO), NaBr, and water; mixing the compound represented by Formula I with the aqueous solution to obtain a mixture; adjusting the pH of the mixture; adding NaClO to the mixture, to thereby oxidize the compound represented by Formula I; adjusting the pH of the mixture after the addition of the NaClO so as to keep the pH of the mixture essentially unchanged; and isolating the oxidized compound so that the oxidation degree of the compound is at least 80 %.

The method may comprise providing an aqueous solution comprising a TEMPO derivative, KBr or NaBr, and water; mixing the compound represented by Formula I with the aqueous solution to obtain a mixture; adjusting the pH of the mixture; adding an oxidizing agent to the mixture, to thereby oxidize the compound represented by Formula I; adjusting the pH of the mixture after the addition of the oxidizing agent so as to keep the pH of the mixture essentially unchanged; and isolating the oxidized compound so that the oxidation degree of the compound is at least 80 %.

The compound represented by Formula I may be oxidized e.g. at a temperature in the range of about 0 - 40 °C, preferably about 10 - 30 °C, more preferably about 20 - 30 °C, even more preferably about 15 - 25 °C, or most preferably about 20 - 25 °C.

The compound represented by Formula I may be oxidized e.g. at a pH in the range of about 7 - 10, or preferably about 8 - 9, or most preferably about 8.3 - 8.4

The compound represented by Formula I may be oxidized e.g. at a temperature in the range of about 10 - 30 °C and at a pH in the range of about 8 - 9, or preferably at a temperature in the range of about 20 - 25 °C and at a pH in the range of about 8 - 9, or most preferably at a temperature in the range of about 20 - 25 °C and at a pH in the range of about 8.3 - 8.4.

The method may comprise providing an aqueous solution comprising a TEMPO derivative, KBr or NaBr, and water; mixing the compound represented by Formula I with the aqueous solution to obtain a mixture; adjusting the pH of the mixture; adding an oxidizing agent to the mixture, to thereby oxidize the compound represented by Formula I; adjusting the pH of the mixture after the addition of the oxidizing agent so as to keep the pH of the mixture essentially unchanged; and isolating the oxidized compound so that the oxidation degree of the compound is at least 80 %, wherein the temperature is in the range of about 20 - 25 °C and the pH is in the range of about 8 - 9.

The molar ratio of the compound represented by Formula I to the TEMPO at the beginning of the oxidizing may be e.g. in the range of 1 - 50, preferably 5 - 30, more preferably 10 - 20, even more preferably 13 - 17, or most preferably about 15.

The molar ratio of the compound represented by Formula I at the beginning of the oxidizing to the NaBr may be e.g. in the range of 1 - 10, preferably 1 - 5, or more preferably 1 - 3, or most preferably about 2.

The mass ratio of the compound represented by Formula I at the beginning of the oxidizing to the NaClO may be e.g. in the range of 1 - 50, preferably 3 - 30, more preferably 6 - 15, even more preferably 8 - 12, even more preferably 9 - 11, or most preferably about 10.

For example, a solution comprising about 4 wt % of the compound represented by Formula I may be prepared. 2,2,6,6-tetramethyl-1-piperidinyloxy free radical (TEMPO) and KBr may be dissolved in distilled water. When the TEMPO is completely dissolved, the TEMPO solution may be mixed with the solution comprising the compound represented by Formula I, and the pH value of the mixture may be adjusted to about 8 - 9, for example using saturated aqueous sodium bicarbonate. An amount of NaClO may then be added to the mixture, and the pH of the solution may be kept essentially unchanged by using a pH-stat method. When a desired oxidation degree is reached, the reaction may be either be stopped by a reducing agent and/or by isolating the oxidized compound from the reaction mixture. Suitable reducing agents are e.g. sodium thiosulfate (e.g. 10% aqueous solution), sodium sulfite (e.g. 15% aqueous solution), or hydroxylammonium chloride. The isolating of the oxidized compound may be performed by precipitation or by chromatography, e.g. by reversed-phase HPLC, medium-pressure column chromatography, or flash chromatography.

For example, a solution comprising about 4 wt % of the compound represented by Formula I may be prepared. 2,2,6,6-tetramethyl-1-piperidinyloxy free radical (TEMPO) and KBr may be dissolved in distilled water. When the TEMPO is completely dissolved, the TEMPO solution may be mixed with the solution comprising the compound represented by Formula I, and the pH value of the mixture may be adjusted to about 8 - 9 using saturated aqueous sodium bicarbonate. An amount of NaClO may then be added to the mixture, and the pH of the solution may be kept essentially unchanged by using a pH-stat method. When a desired oxidation degree is reached, the reaction may be either be stopped by a sodium thiosulfate, sodium sulfite, or hydroxylammonium chloride.

The compound represented by Formula I may be a compound represented by Formula II 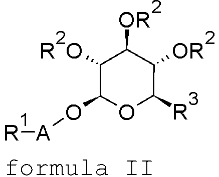 wherein R¹, R², and R³ are as defined above and/or in one or more embodiments described in this specification; and A is selected from A¹, A², A³, A⁴, and A⁵ as set out above.

A may, in some embodiments, be A¹ or A². In other words, A may be selected from A¹ and A². Such a compound represented by Formula I is relatively small, which may render it easier to purify after the oxidation.

A may, in some embodiments, be A¹, A² or A³. In other words, A may be selected from A¹, A² and A³.

A may, in some embodiments, be A².

The method may further comprise adding one or more amino acid residues of the auristatin to the oxidized compound, thereby forming the auristatin moiety, such that a uronyl-monomethylauristatin compound is obtained. In other words, the method may further comprise forming a uronyl-monomethylauristatin compound from the oxidized compound. The uronyl-monomethylauristatin compound may be understood as referring to a compound represented by Formula I, wherein R³ is COOH and A is A⁵.

The auristatin moiety may be understood as referring to the following moiety:

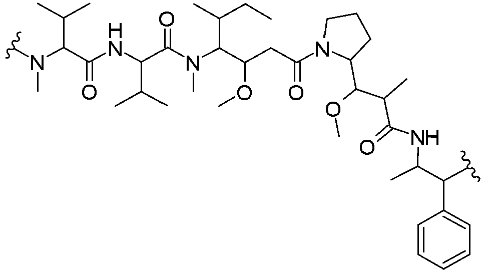

As a skilled person will understand, the term "amino acid residue" does not in this context necessarily refer to the 21 (or 22) proteinogenic amino acid residues. Instead, it refers to the amino acid residues making up the above auristatin moiety. The one or more amino acid residues may be added in an order in which they may make up the above auristatin moiety, either separately or in one or more larger moieties. Auristatin moieties may be prepared according to the general methods described in U.S. Pat. No. 5,635,483; U.S. Pat. No. 5,780,588; Pettit et al., 1989, J. Am. Chem. Soc. 111:5463-5465; Pettit et al., 1998, Anti-Cancer Drug Design 13:243-277; and Pettit et al., 1996, J. Chem. Soc. Perkin Trans. 15:859-863.

In the context of this specification, the term "moiety" may refer to a specified segment, fragment, or functional group of a molecule or compound. Chemical moieties are sometimes indicated as chemical entities that are embedded in or appended to (i.e., a substituent or variable group) a molecule, compound or chemical Formula.

The one or more amino acid residues of the auristatin moiety may be added in one or more steps. For example, in an embodiment in which A is A², the compound represented by Formula I may be reacted with the following compound: 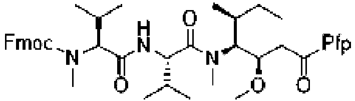 The product may be reacted e.g. with diisopropylamine to remove the Fmoc protecting group and to thereby yield the uronyl-monomethylauristatin compound. Such a reaction is shown in detail in Example 1.

For example, the compound represented by Formula I may be reacted with a compound comprising the moiety represented by B¹, B², B³ or B⁴:
In embodiments in which A is A¹, the compound represented by Formula I may be reacted with a compound comprising the moiety represented by B¹

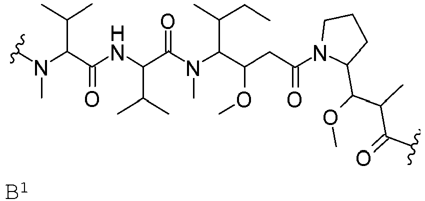

In embodiments in which A is A², the compound represented by Formula I may be reacted with a compound comprising the moiety represented by B²

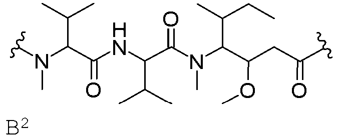

In embodiments in which A is A³, the compound represented by Formula I may be reacted with a compound comprising the moiety represented by B³

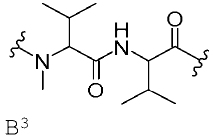

In embodiments in which A is A⁴, the compound represented by Formula I may be reacted with a compound comprising the moiety represented by B⁴

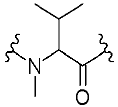

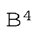

A compound represented by Formula I is also disclosed: 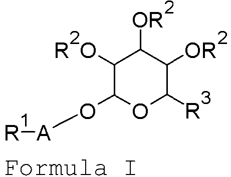
wherein R¹ is either H or a protecting group;
A is selected from A¹, A², A³, A⁴, and A⁵ 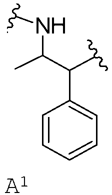 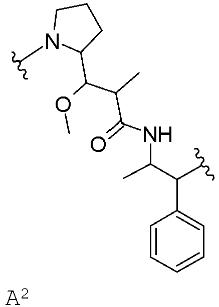 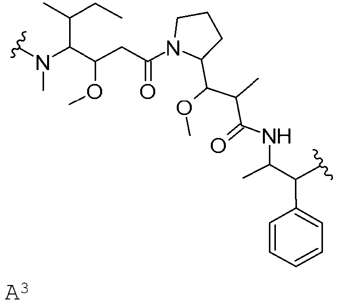 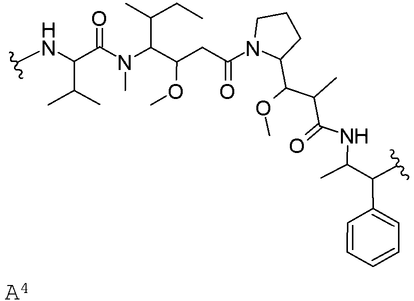 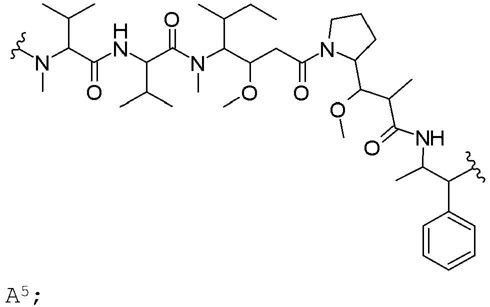
R³ is OH, CH₂OH, CH₂OR² or COOH; and
each R² is independently selected from either H or a protecting group.
R³ may be CH₂OH, CH₂OR² or COOH.
R³ may be CH₂OH or COOH.

In embodiments in which A is A¹ or A², and R³ is OH, CH₂OH, CH₂OR² or COOH, the compound represented by Formula I may be purified to be essentially stereochemically pure. The purification of the stereochemically essentially pure compound may be performed by crystallization or by chromatography, e.g. by reversed-phase HPLC, medium-pressure column chromatography, or flash chromatography. The stereochemically pure compound may be understood to contain less than 5 %, 4 %, 3 %, 2%, 1 %, 0.5 %, 0.2 %, or 0.1 %, or about 0 % detectable contaminating diastereomers.

Any embodiments described in the context of the method may be understood as being disclosed in the context of the compound, and vice versa.

The protecting group in R¹ may be Boc, i.e. a tert-butyloxycarbonyl protecting group. The Boc group can be added to amines under aqueous conditions using di-tert-butyl dicarbonate in the presence of a base, such as sodium hydroxide. For example, the protecting group which may be R¹ may be selected from Boc, PNZ, and Fmoc.

The protecting group in R² may be Bz or 2,5-difluoro-Bz, i.e. a benzoyl group or a 2,5-difluorobenzoyl group. In some embodiments, each R² may be independently selected from H, Bz, and 2,5-difluoro-Bz. In some embodiments, each R² may be independently selected from H and Bz or from H and 2,5-difluoro-Bz. In some embodiments, each R² may be H. In some embodiments, each R² may be Bz. In some embodiments, each R² may be 2,5-difluoro-Bz.

In some embodiments of the compound, R³ may be OH, CH₂OR² or CH₂OH. In some embodiments of the compound, R³ may be CH₂OR² or CH₂OH. Any references to the "compound represented by Formula I (or II) at the beginning of the oxidizing" may be understood as referring to a compound represented by Formula I (or II) in which R³ is OH, CH₂OR² or CH₂OH (or CH₂OR² or CH₂OH, in particular CH₂OH).

When R³ is CH₂OR², the R² in R³ being CH₂OR² (i.e. the protecting group attached to the primary hydroxyl) may be a protecting group. It may be any protecting group described in this specification. Each R², including the R² in R³, may be independently selected. In some embodiments, the R² in R³ may be the same protecting group as the other instances of R² (i.e. the R² groups attached to the secondary hydroxyls). In some embodiments, the R² in R³ may be a protecting group different from the protecting group(s) represented by other instances of R².

In some embodiments, R¹ is Boc or Fmoc. In some embodiments, R¹ is Boc. In some embodiments, R¹ is Fmoc.

In some embodiments, R³ is CH²OH or CH²OR².

In some embodiments, each R² is Bz or H. In some embodiments, each R² is Bz. In some embodiments, each R² is H.

In an embodiment, R³ is CH²OH or CH²OR²; R¹ is Boc or Fmoc; and each R² is Bz or H.

In an embodiment, R³ is CH²OH or CH²OR²; R¹ is Boc; and each R² is Bz or H.

In an embodiment, R³ is CH²OH or CH²OR²; R¹ is Fmoc; and each R² is Bz or H.

In an embodiment, R³ is CH²OH or CH²OR²; R¹ is Boc or Fmoc; and each R² is Bz.

In an embodiment, R³ is CH²OH or CH²OR²; R¹ is Boc or Fmoc; and each R² is H.

In an embodiment, R³ is CH²OR²; R¹ is Boc or Fmoc; and each R² is Bz or H.

In an embodiment, R³ is CH²OR²; R¹ is Boc; and each R² is Bz or H.

In an embodiment, R³ is CH²OR²; R¹ is Fmoc; and each R² is Bz or H.

In an embodiment, R³ is CH²OR²; R¹ is Boc or Fmoc; and each R² is Bz.

In an embodiment, R³ is CH²OR²; R¹ is Boc or Fmoc; and each R² is H.

In an embodiment, R³ is CH²OH; R¹ is Boc or Fmoc; and each R² is Bz or H.

In an embodiment, R³ is CH²OH; R¹ is Boc; and each R² is Bz or H.

In an embodiment, R³ is CH²OH; R¹ is Fmoc; and each R² is Bz or H.

In an embodiment, R³ is CH²OH; R¹ is Boc or Fmoc; and each R² is Bz.

In an embodiment, R³ is CH²OH; R¹ is Boc or Fmoc; and each R² is H.

In an embodiment, R³ is CH²OH or CH²OR²; R¹ is Boc or Fmoc; each R² is Bz or H; and A is A¹ or A².

In an embodiment, R³ is CH²OH or CH²OR²; R¹ is Boc; each R² is Bz or H; and A is A¹ or A².

In an embodiment, R³ is CH²OH or CH²OR²; R¹ is Fmoc; each R² is Bz or H; and A is A¹ or A².

In an embodiment, R³ is CH²OH or CH²OR²; R¹ is Boc or Fmoc; each R² is Bz; and A is A¹ or A².

In an embodiment, R³ is CH²OH or CH²OR²; R¹ is Boc or Fmoc; each R² is H; and A is A¹ or A².

In an embodiment, R³ is CH²OR²; R¹ is Boc or Fmoc; each R² is Bz or H; and A is A¹ or A².

In an embodiment, R³ is CH²OR²; R¹ is Boc; each R² is Bz or H; and A is A¹ or A².

In an embodiment, R³ is CH²OR²; R¹ is Fmoc; each R² is Bz or H; and A is A¹ or A².

In an embodiment, R³ is CH²OR²; R¹ is Boc or Fmoc; each R² is Bz; and A is A¹ or A².

In an embodiment, R³ is CH²OR²; R¹ is Boc or Fmoc; each R² is H; and A is A¹ or A².

In an embodiment, R³ is CH²OH; R¹ is Boc or Fmoc; each R² is Bz or H; and A is A¹ or A².

In an embodiment, R³ is CH²OH; R¹ is Boc; each R² is Bz or H; and A is A¹ or A².

In an embodiment, R³ is CH²OH; R¹ is Fmoc; each R² is Bz or H; and A is A¹ or A².

In an embodiment, R³ is CH²OH; R¹ is Boc or Fmoc; each R² is Bz; and A is A¹ or A².

In an embodiment, R³ is CH²OH; R¹ is Boc or Fmoc; each R² is H; and A is A¹ or A².

The compound represented by Formula I may be a compound selected from the following:

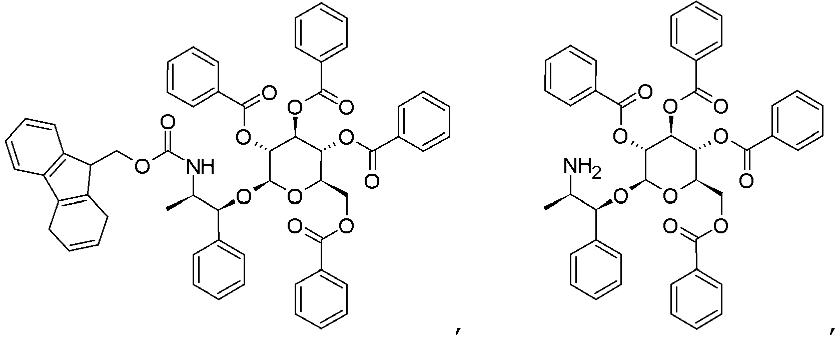

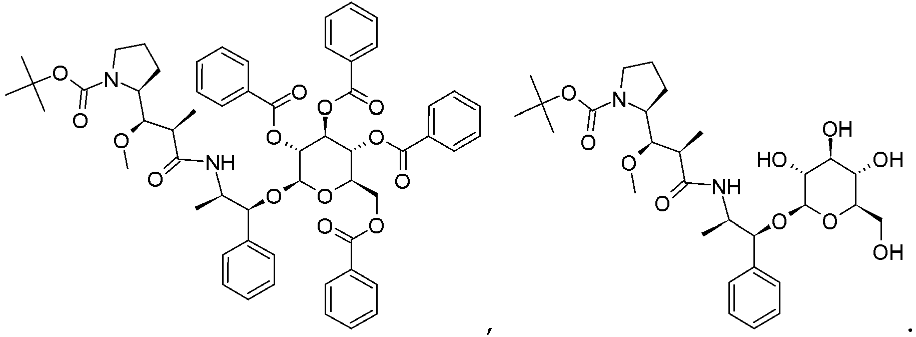

In some embodiments of the compound, R³ may be COOH. Any references to the "oxidized compound" may be understood as referring to a compound represented by Formula I or II in which R³ is COOH.

In some embodiments of the compound, R³ is CH₂OH or CH₂OR², wherein R² is a protecting group.

The compound may be represented by Formula II 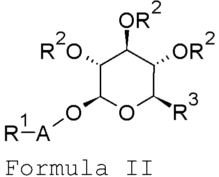 wherein R¹, R², R³ and A are as defined in the context of Formula I.

The compound may be represented by e.g. any one of the formulas below: 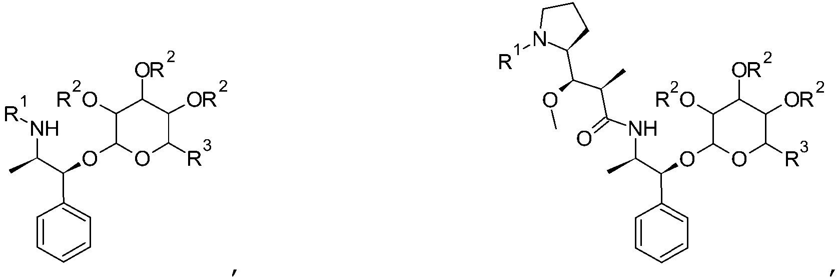 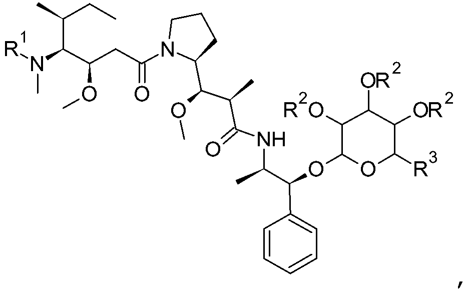 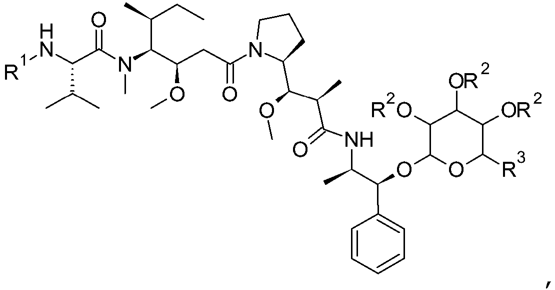 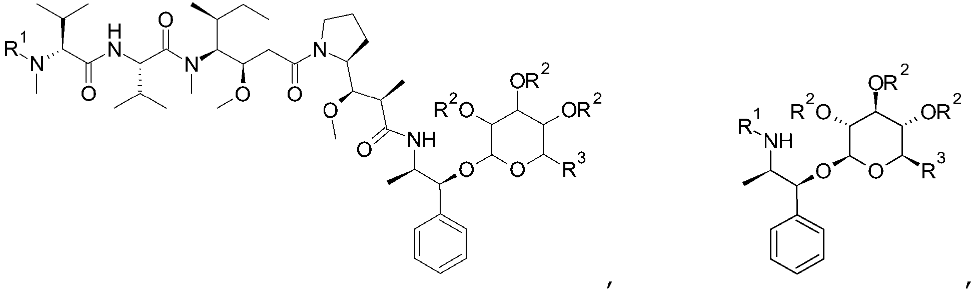 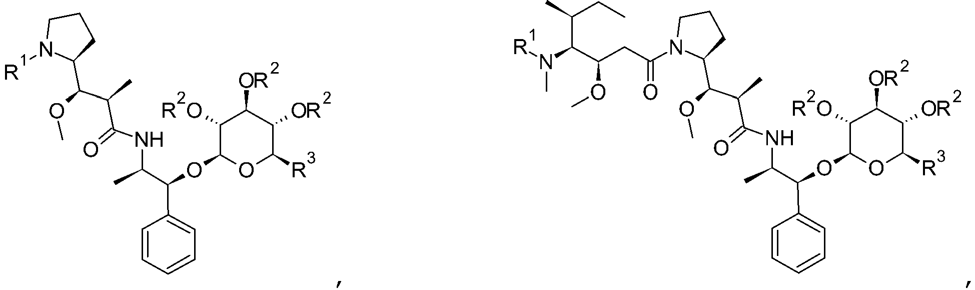 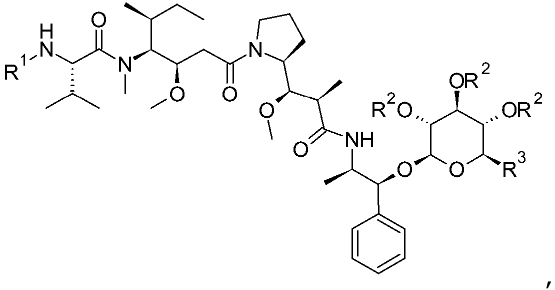 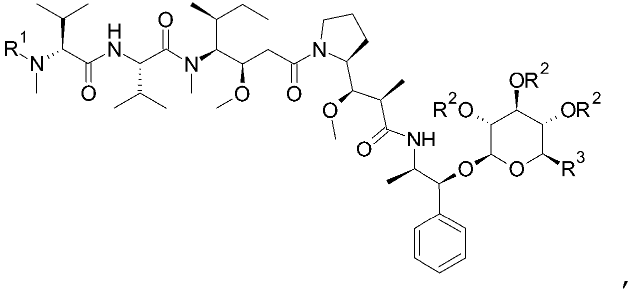 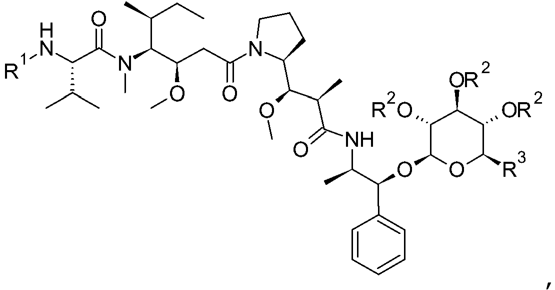 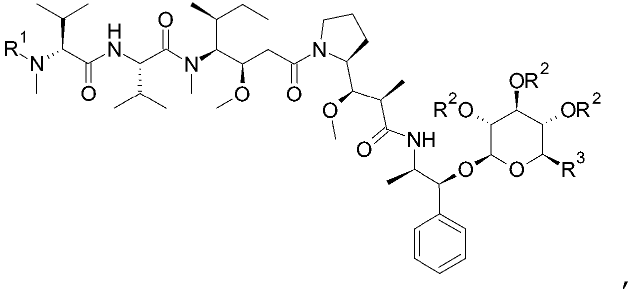 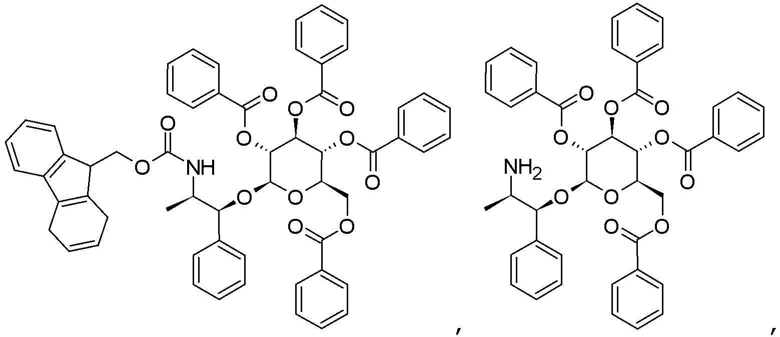 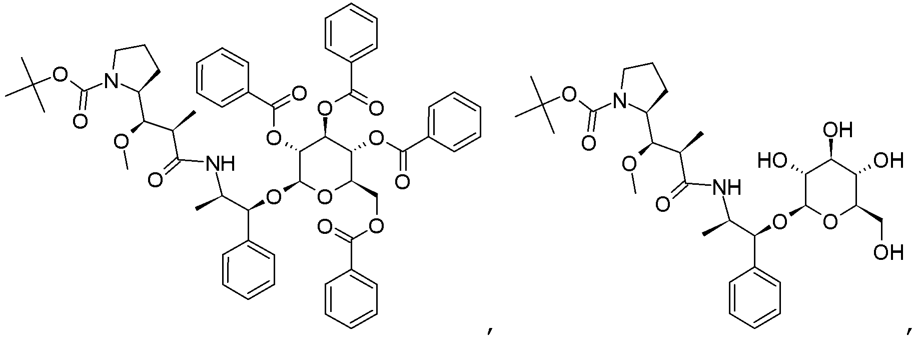 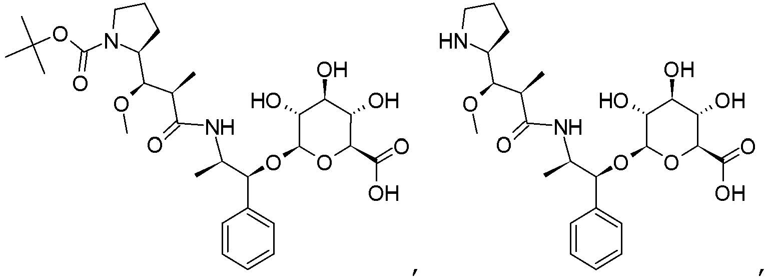 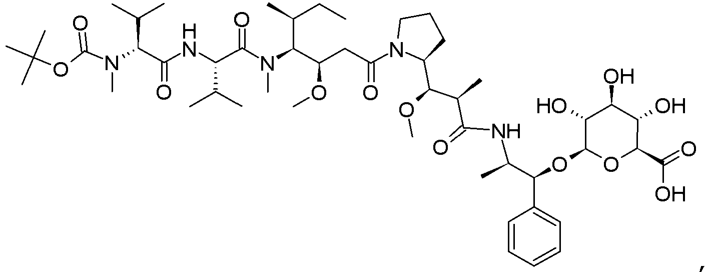 or 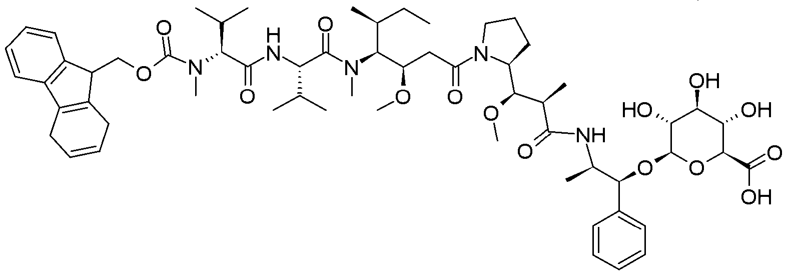 wherein R¹, R², and R³ are according to one or more embodiments disclosed in this specification (e.g. as defined above).

With the present method, yield of the method may be significantly improved, for example as compared to methods in which the uronyl (or glucuronyl) moiety is added into MMAE or to a fragment thereof. Further, stereochemistry of the resulting compounds may be easier to control. The compound represented by Formula I may also be smaller than MMAE or MMAU, such that the resulting oxidized compound may be easier to purify.

The compounds according to one or more embodiments described in this specification may be conjugated further, e.g. to a linker and/or to a targeting unit, such as an antibody.

The method may further comprise
conjugating the uronyl-monomethylauristatin compound to a linker segment, thereby obtaining a linker-payload conjugate; and
optionally conjugating the linker-payload conjugate to a targeting unit.

The method may thus be a method for preparing a linker-payload conjugate and/or a targeting unit-linker-payload conjugate. The linker-payload conjugate thus comprises a uronyl-monomethylauristatin moiety derived from the uronyl-monomethylauristatin compound.

The payload may here be understood as referring to MMAE or MMAU.

The targeting unit may be e.g. an antibody or a fragment thereof.

In other words, the uronyl-monomethylauristatin compound obtained by the method according to one or more embodiments described herein may be incorporated into a linker-payload conjugate comprising the uronyl-monomethylauristatin moiety operably connected to a linker segment suitable for conjugation to a targeting unit.

Synthetic methods useful for the preparation of linker segments are described in Schemes of Examples 2-6. Methods for attaching linkers to payload moieties are described, for example, in US20050238649 and US20060024317.

The linker-payload conjugates may be prepared using a linker segment comprising two or more reactive sites for binding to the uronyl-monomethylauristatin moiety and to a targeting unit such as an antibody. In certain embodiments, the linker segment comprises a reactive site containing an electrophilic group capable of reacting with a nucleophilic group present on the targeting unit. Suitable nucleophilic groups on an antibody include, but are not limited to, sulfhydryl groups, which may react with an electrophilic group on the linker segment to form a covalent bond. Suitable electrophilic groups include e.g. maleimide groups, which may provide a convenient site for antibody attachment.

In certain embodiments, the linker segment comprises a para-aminobenzyl (PAB) spacer capable of functioning as a self-immolative spacer. Such a self-immolative spacer may be capable of undergoing fragmentation following cleavage of an upstream cleav-able unit, thereby releasing the uronyl-monomethylauristatin moiety.

In some embodiments, the linker segment comprises one or more amino acid residues selected from β-alanine, valine, serine and Serine(β-Glc). The linker segment may comprise a conjugation group. Such a conjugation group may function as the reactive site described above. In particular embodiments, the linker segment comprises a β-Ala-Val-Ser(β-Glc) sequence positioned between the conjugation group and the PAB spacer.

In certain embodiments, the linker segment comprises a conjugation group, which is a maleimide-containing group configured to react with thiol groups, such as a cysteine residue present on the targeting unit. In some embodiments, the linker-payload conjugate comprises a maleimidoacetyl group connected to a β-alanine residue optionally connected to a valine residue and further connected to a PAB spacer, the PAB spacer being operably linked to the uronyl-monomethylauristatin moiety (derived from the uronyl-monomethylauristatin compound).

The method may comprise one or more sequential peptide coupling reactions, one or more activation steps, and one or more protecting group manipulations performed under conditions selected to preserve the structural integrity of the auristatin moiety.

The linker segment may comprise a further glucosyl moiety. The further glucosyl moiety may also be oxidized to a glucuronyl moiety.

In certain embodiments, protecting groups selected from benzoyl, substituted benzoyl, 2,5-difluorobenzoyl, Boc, Fmoc, or para-nitrobenzyloxycarbonyl (PNZ) may be used during synthesis of the linker-payload conjugate. Benzoyl protecting groups may be employed to promote selective formation of the β-anomer during glycosylation reactions, whereas acetyl protecting groups may be avoided due to instability under subsequent reaction conditions.

In some embodiments, 2,5-difluorobenzoyl protecting groups are removable under controlled basic conditions while substantially reducing β-elimination side reactions of the glucoside. Saponification may be carried out to remove acyl protecting groups from the further glucosyl moiety, for example using a methoxide base in an alcohol solvent, wherein base concentration, temperature, and reaction time are selected to minimize elimination of the glycosidic linkage.

In certain embodiments, β-elimination of the glucoside during saponification is reduced to less than 20%, less than 10%, less than 5%, or less than 2% of total product, thereby substantially preserving the β-configuration of the further glucosyl moiety.

Amine protecting groups may be removed under conditions selected to avoid cleavage of adjacent linker components. In some embodiments, Boc protecting groups are removed under acidic conditions selected to limit cleavage of the PAB spacer. In certain embodiments, PNZ protecting groups are removed under reductive conditions, for example using zinc-mediated or formate-mediated reduction.

In some embodiments, oxidation of the further glucosyl moiety of the linker segment to a glucuronyl moiety is performed following selected deprotection steps. In certain embodiments, oxidation is performed prior to assembly of the complete auristatin moiety, after partial assembly of the auristatin moiety but prior to linker attachment, or after formation of the full linker-payload conjugate. In some embodiments, saponification and oxidation are performed sequentially, and the order of these steps is selected to minimize degradation of the further glucosyl moiety.

In certain embodiments, oxidation of the further glucosyl moiety present in a linker segment is carried out using a catalytic oxidation system comprising a catalyst, such as TEMPO, and an oxidizing agent, such as a hypochlorite oxidizing agent. The oxidizing agent may be any oxidizing agent described in this specification. The catalyst may be any catalyst described in this specification. The further glucosyl moiety may be oxidized in the presence of further components, such as a reaction accelerator. The reaction accelerator may be any reaction accelerator described in this specification.

In some embodiments, oxidation of the further glucosyl moiety of the linker segment is performed in an aqueous medium in the presence of sodium hypochlorite and a bromide salt under controlled pH conditions selected to selectively convert a primary hydroxymethyl group of the further glucosyl moiety to a carboxyl group.

In certain embodiments, additional oxidizing components selected from sodium chlorite or related oxidizing species are used to facilitate conversion of the further glucosyl moiety to a glucuronyl moiety.

In some embodiments, oxidation of the further glucosyl moiety of the linker segment is performed prior to attachment of the auristatin moiety. Thus a linker segment comprising a further uronyl moiety may be obtained.

In other embodiments, oxidation of the further glucosyl moiety is performed in the presence of the uronyl-auristatin moiety under conditions selected to maintain the structural integrity of the auristatin moiety.

In certain embodiments, the oxidation selectively converts the primary hydroxymethyl group of the further glucosyl moiety to a carboxyl group while substantially avoiding oxidation of secondary hydroxyl groups, such that less than 10%, less than 5%, or less than 1% of secondary hydroxyl groups are oxidized.

In some embodiments, the oxidation proceeds with a conversion of at least 80%, at least 90%, at least 95%, or at least 98%, and the resulting linker segment comprising the resulting further uronyl moiety is obtained with a chemical purity of at least 90%, at least 95%, at least 98%, or at least 99% following chromatographic purification.

In certain embodiments, the oxidation conditions are compatible with any amide bonds, carbamate groups, peptide linkages, and self-immolative spacer units present in the linker-payload conjugate.

In certain embodiments, the linker-payload conjugate comprises a maleimidoacetyl-β-Ala-Val-Ser(Glc)-PAB linker segment conjugated to the uronyl-monomethylauristatin moiety according to one or more embodiments described in this specification.

In certain embodiments, the linker-payload conjugate comprises a maleimidoacetyl-β-Ala-Val-Ser (GlcA) -PAB linker segment comprising the uronyl-monomethylauristatin moiety. The linker-payload conjugate may thus be a maleimidoacetyl-β-Ala-Val-Ser(GlcA)-PAB-MMAU conjugate.

In some embodiments, the resulting linker-payload conjugate is purified by chromatographic methods. A product of high chemical purity may thus be obtained. The resulting linker-payload conjugate is suitable for conjugation to the targeting unit selected from an antibody, an antibody fragment, an antigen-binding fragment, a fusion protein, a peptide ligand, or another targeting molecule capable of selectively binding to a target.

In certain embodiments, the linker-payload conjugate is configured for use in an antibody-drug conjugate.

### EXAMPLES

Reference will now be made in detail to various embodiments, an example of which is illustrated in the accompanying drawings.

The description below discloses some embodiments in such a detail that a person skilled in the art is able to utilize the embodiments based on the disclosure. Not all steps or features of the embodiments are discussed in detail, as many of the steps or features will be obvious for the person skilled in the art based on this specification.

General equipment and reagents include:
HPLC: Äkta Purifier 100 and 10 (GE Healthcare)
HiTrap MabSelect SuRe protein A column 1 ml (GE Healthcare)
HiTrap Desalting column 5 ml (GE Healthcare)
Loading buffer: 20 mM Na-phosphate pH 7.2, 150 mM NaCl, or PBS (Lonza)
Elution buffer: 0.1 M citrate pH 3.0
Desalting buffer: PBS, Gibco
Spectrophotometer: Nanodrop one (Thermo Fisher Scientific)
TCEP: Tris-2-carboxyethyl phosphine hydrochloride
DMSO: Dimethyl sulfoxide
TFA: Trifluoroacetic acid
DHAP: 2', 5', dihydroxyacetophenone
BSA: Bovine serum albumin
MALDI-TOF mass spectrometer: Bruker Ultraflex III
Superdex 200 column (10 x 300 mm, GE Healthcare)
TSKgel ButylNPR column (4.6 mm x 3.5 cm, Tosoh Biosciences)
PLRP-S column (1000 Å, 8µM, 150x2.1 mm, Agilent)
SDS-PAGE: 4-15% Mini-Protean TGX gel (Bio-Rad), Laemmli running buffer, Spectra multicolor broad range protein ladder (Thermo Scientific), Imperial protein stain (Pierce Thermo Scientific).

### EXAMPLE 1 - Synthesis of MMAU payload (GLD-10)

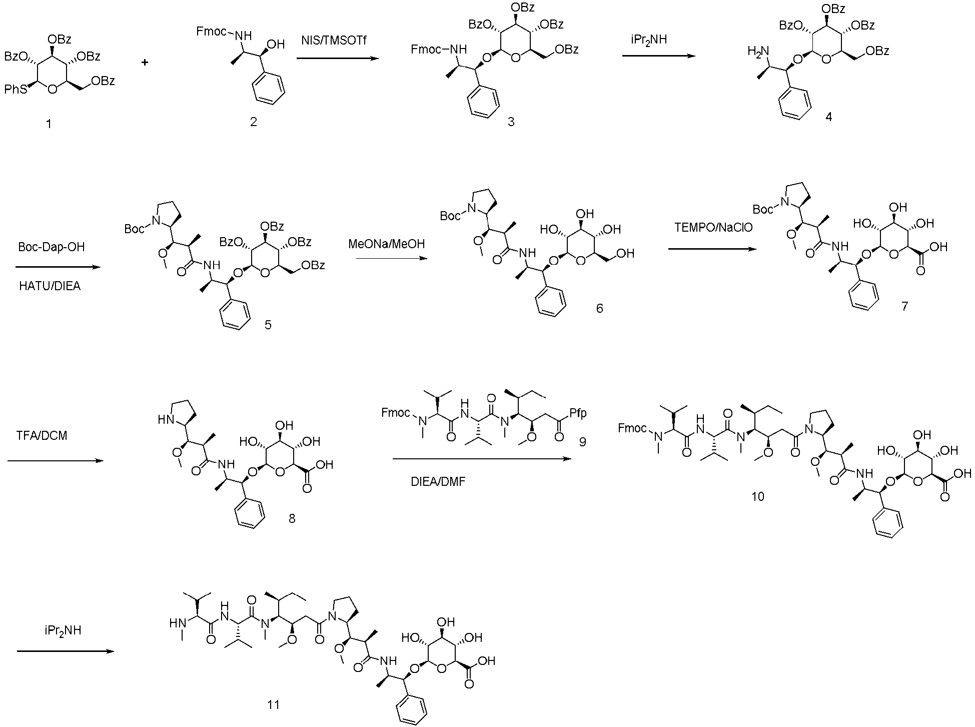

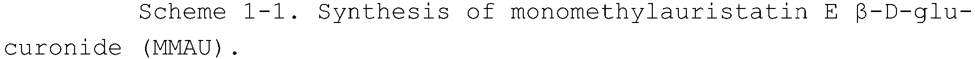

To a stirred solution of thioglycoside **1** (4.13 g) and compound **2** (1.86 g) in anhydrous DCM (100 mL) under Argon atmosphere at 0 °C (ice bath) was added NIS (1.48 g), followed by TMSOTf (0.1 mL) and the reaction mixture was stirred at room temperature for 30 mins. The mixture was diluted with DCM (200 mL) and washed with Na₂S₂O₃ (10%, aq., 100 mL) and water (100 mL). The organic layer was concentrated under reduced pressure to dryness. The residue obtained was dissolved in DMF (30 mL) and diisopropylamine (30 mL) was added. The mixture was stirred at room temperature for 3 h and then concentrated to about 25 mL under reduced pressure. To this solution was added anhydrous DMF (40 mL), Boc-Dap-OH (1.44 g, 5 mmol) and DIEA (1.75 mL, 10 mmol), followed by HATU (1.9 g, 5 mmol). The mixture was stirred at room temperature for 15 min and then diluted with EtOAc (200 mL). The mixture was washed successively with 0.5 N hydrochloric acid (200 mL), water (200 mL), saturated aq. NaHCO₃ (100 mL), and water (200 mL). The organic phase was dried (over Na₂SO₄) and evaporated to dryness under reduced pressure. The crude product was purified by column chromatography (silica gel, 220 g column, EtOAc/hexane: 1/2 to 2/1 in 60 mins, flow rate: 40 mL/min) to give compound **5** as a white foam (4.3 g, 86% over three steps).

Compound **5** (4 g, 4 mmol) was dissolved in anhydrous MeOH (100 mL) and MeONa (25% in MeOH, Sigma-Aldrich, 0.5 mL) was added. The mixture was stirred at room temperature for 2 h and then HCl (4M solution in dioxane, 0.55 mL) was added. The solvents were removed under reduced pressure and the residue was dissolved in acetonitrile/water (2/1, v/v, 30 mL). To this mixture was added KBr (1M aq. solution, 2 mL), TEMPO (44 mg, 0.28 mmol), and saturated aq. NaHCO₃ (6 mL), followed by bleach (commercial grade from Costco, 9 mL). The reaction was stirred at room temperature for 15 mins and more saturated aq. NaHCO₃ (6 mL) and bleach (9 mL) were added. After stirring at room temperature for 1 h, the mixture was purified directly by preparative RP-HPLC to give compound **7** as a white solid (2.1 g, 3.3 mmol, 82.5% over two steps) after lyophilization.

### Preparative HPLC method details:

Column: Phenomenex Gemini NX 5m, C18, 110 Å, 150 x 50 mm
Instrument: Shimadzu LC with CTC IFC
Mobile phase: A) water (0.1% TFA), B) acetonitrile
Gradient: 10-60% B over 20 min, flow 50 mL/min

The desired compound eluted at 18 min

Compound **7** (2.1 g) was treated with TFA/DCM (1/4, v/v, 50 mL) at room temperature for 30 mins and the reaction was diluted with toluene (50 mL). The mixture was evaporated to dryness under reduced pressure. The obtained compound **8** (TFA salt) was used directly in the next step without further purification.

Compound **8** (TFA salt, 176 mg) was dissolved in anhydrous DMF (2 mL) and DIEA (0.2 mL) was added, followed by the Pfp ester (compound **9,** 250 mg). The reaction mixture was stirred at room temperature (22 °C) for 2 h and then 2 mL of diisopropylamine was added. After 3 h, the reaction mixture was concentrated to about 2 mL under reduced pressure and the residue was purified by prep RP-HPLC to give compound **11** (GLD-10, MMAU) as a white powder (221 mg, 76%) after lyophilization.

### Preparative HPLC method details:

Column: Phenomenex Gemini NX 5m, C18, 110 Å, 150 x 50 mm
Instrument: Shimadzu LC with CTC IFC
Mobile phase: A) water (0.1% TFA), B) acetonitrile
Gradient: 5-40% B over 20 min, flow 50 mL/min

The desired compound eluted at 19 min

### EXAMPLE 2 - Synthesis of mavg-MMAU linker-payload (GLD-22), procedure A

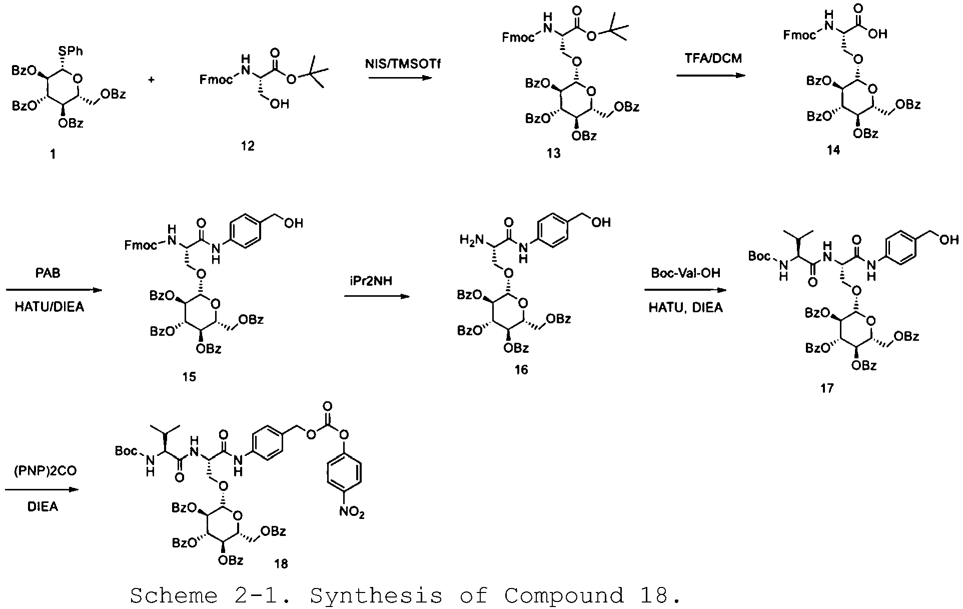

To a stirred solution of thioglycoside **1** (7.1 g) and compound **12** (3.7 g) in anhydrous DCM (200 mL) under argon atmosphere at 0 °C was added NIS (2.5 g), followed by TMSOTf (0.1 mL) and the solution was stirred at room temperature for 10 mins. The mixture was diluted with DCM (200 mL) and washed with Na₂S₂O₃ (10%, aq, 100 mL x 2) and water (50 mL). The organic layer was concentrated under reduced and the residue was redissolved in TFA/DCM (1/1, v/v, 100 mL). After 30 min, the mixture was diluted with DCM (200 mL) and washed with water (100 mL x 4). The organic layer was evaporated to dryness to give the crude acid **14,** which was dissolved in DMF (100 mL). To this solution, p-aminobenzylalcohol (1.3 g) was added, followed by DIEA (3.6 mL) and HATU (3.9 g). The reaction was stirred at room temperature for 20 mins, and then diluted with EtOAc (300 mL). The mixture was washed with 0.5 M hydrochloric acid (150 mL) and water (200 mL). The organic layer was dried (over Na₂SO₄) and evaporated to dryness under reduced pressure. The residue was triturated with hexane/diethyl ether (1/1, 100 mL) to give the crude compound **15** as a tan solid (9.8 g), which was dissolved in DMF (50 mL). Diisopropylamine (50 mL) was added, and the mixture was stirred at room temperature for 2 h. The reaction was then concentrated under reduced pressure to about 40 mL and diluted with DMF (60 mL). Boc-Val-OH (2.17 g) and DIEA (3.5 mL) were added, followed by HATU (3.8 g), and the mixture was stirred at room temperature. After 20 min, the reaction was diluted with EtOAc (300 mL). The mixture was washed with 0.5 M hydrochloric acid (150 mL) and water (200 mL). The organic layer was dried (over Na₂SO₄) and evaporated to dryness under reduced pressure. The crude product was purified by column chromatography (silica gel, 220 g column x 2, EtOAc/hexane: 1/2 to 2/1 in 60 mins, flow rate: 40 mL/min) to give compound **17** as a pale-yellow solid (6.5 g, 66 % over five steps).

Compound **17** (6.5 g) was dissolved in DMF (50 mL). To this solution, DIEA (1.4 mL) and bis PNP carbonate (3 g) were added, and the mixture was stirred at room temperature for 16 h. The reaction was then diluted with EtOAc (300 mL) and washed with water (3 x 100 mL). The organic layer was dried (over Na₂SO₄) and evaporated to dryness under reduced pressure. The residue was purified by column chromatography (silica gel, 220 g column, EtOAc/hexane: 1/3 to 1/1 in 40 mins, flow rate: 40 mL/min) to give compound **18** as a white solid (6.4 g, 84%).

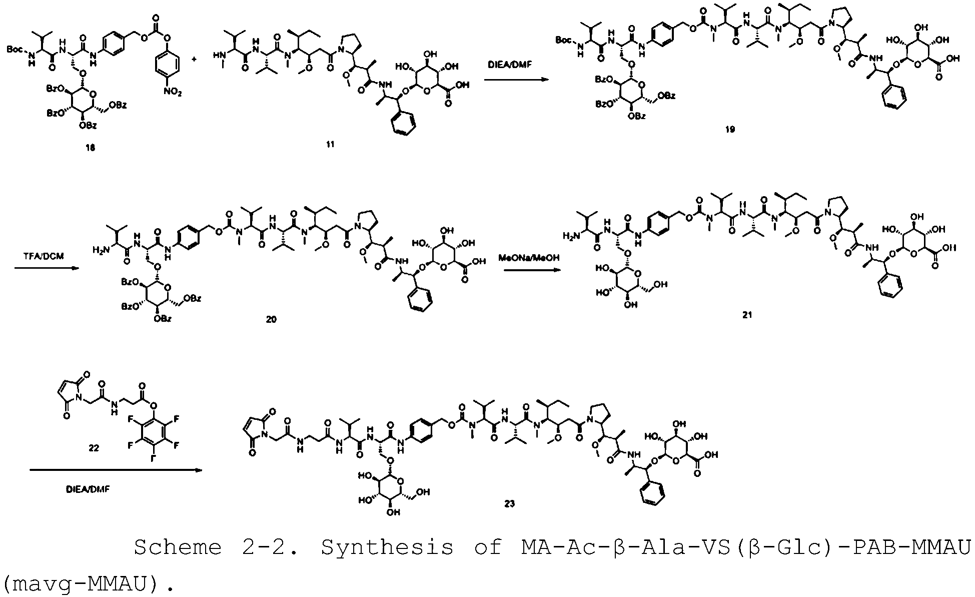

To a solution of compound **18** (4 g) and compound **11** (TFA salt, 3.5 g) in DMF (50 mL) was added DIEA (2.4 mL) and the reaction was stirred at room temperature for 48 h. The mixture was then diluted with EtOAc (250 mL) and washed with hydrochloric acid (0.5 M, 100 mL). The aqueous layer was reextracted with EtOAc (4 x 50 mL) and the combined organic layer was washed with water (100 mL) , dried (over Na₂SO₄) and evaporated to dryness under reduced pressure. The residue was treated with TFA/DCM (1/4, v/v, 50 mL) at room temperature for 90 mins and the mixture was diluted with toluene (50 mL) and evaporated to dryness under reduced pressure. The residue was triturated with diethyl ether (100 mL) and the precipitated solid was collected by filtration. The crude product obtained was redissolved in MeOH (30 mL) and MeONa (4.4 M in MeOH, 2 mL) was added. The reaction was stirred at room temperature for 1 h and then neutralized with 1 N hydrochloric acid (4 mL). The mixture was purified directly by RP-HPLC to give compound **21** as a white solid (TFA salt, 2.4 g, 46% over three steps).

### Preparative HPLC method details:

Column: Phenomenex Gemini NX 5µ, C18, 110 Å, 150 x 50 mm
Instrument: Shimadzu LC with CTC IFC
Mobile phase: A) water (0.1% TFA), B) acetonitrile
Gradient: 5-45% B over 20 min, flow 50 mL/min

The desired compound eluted at 20 min

Compound **21** (2.06 g) was dissolved in DMF (20 mL), and compound **22** (0.54 g) was added, followed by DIEA (0.72 mL). The mixture was stirred at room temperature for 2 h and purified directly by RP-HPLC to give mavg-MMAU (GLD-22, compound **23)** as a white solid (2.1 g, 96%) after lyophilization.

### Preparative HPLC method details:

Column: Phenomenex Gemini NX 5µ, C18, 110 Å, 150 x 50 mm
Instrument: Shimadzu LC with CTC IFC
Mobile phase: A) water (0.1% TFA), B) acetonitrile
Gradient: 10-60% B over 20 min, flow 50 mL/min

The desired compound eluted at 18 min

### EXAMPLE 3 - Improvement of saponification and Boc removal

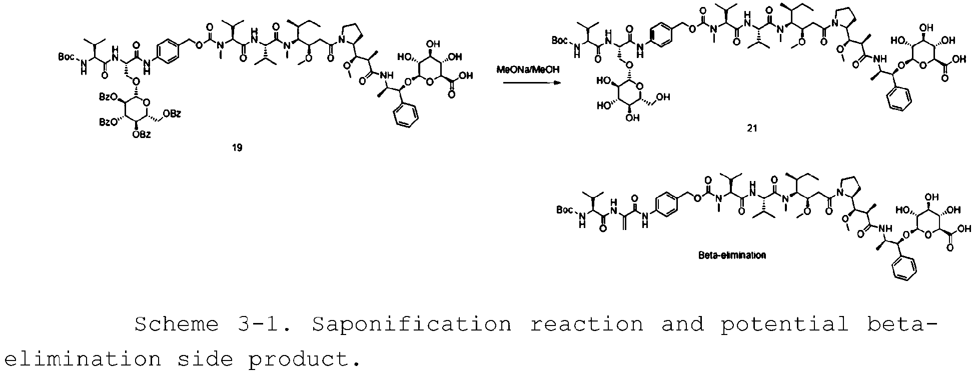

A potential side product was observed during the saponification reaction from compound 19 to 21, namely up to 30% beta-elimination of the glucoside. A bulky protecting group (Bz) was essential to ensure exclusive formation of the beta-glucose anomer, while Ac resulted in non-satisfactory glycosylation reaction and was not stable under the Fmoc removal conditions. When 2,5-difluorobenzoyl was used instead of Bz, it was removed in 10 minutes without beta-elimination being observed.

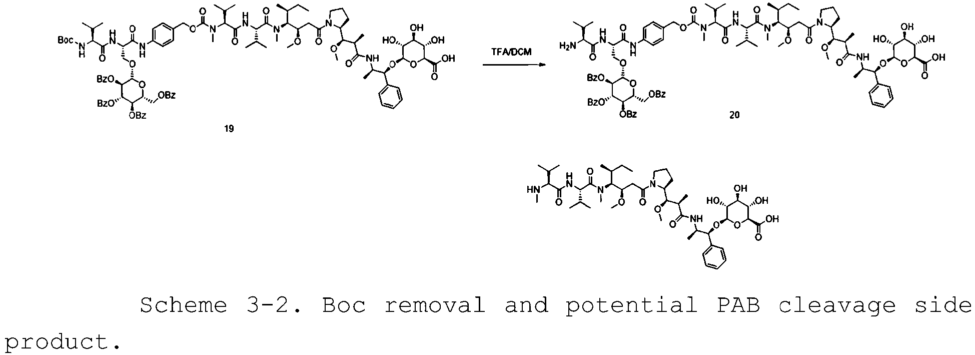

A potential side product was observed during the Boc removal, namely up to 10-15% PAB cleavage. The best condition was to treat the compound with 20% TFA/DCM and monitor the reaction until >90% of the starting material was consumed. If Fmoc was used, it was partially cleaved already in the preceding steps. However, when p-nitrobenzyloxycarbonyl (PNZ) was used to replace Boc, it could be successfully removed with either Zn/formic acid or ammonium formate/MeOH, and no linker cleavage was observed.

### EXAMPLE 4 - Synthesis of mavg-MMAU linker-payload (GLD-22), procedure B

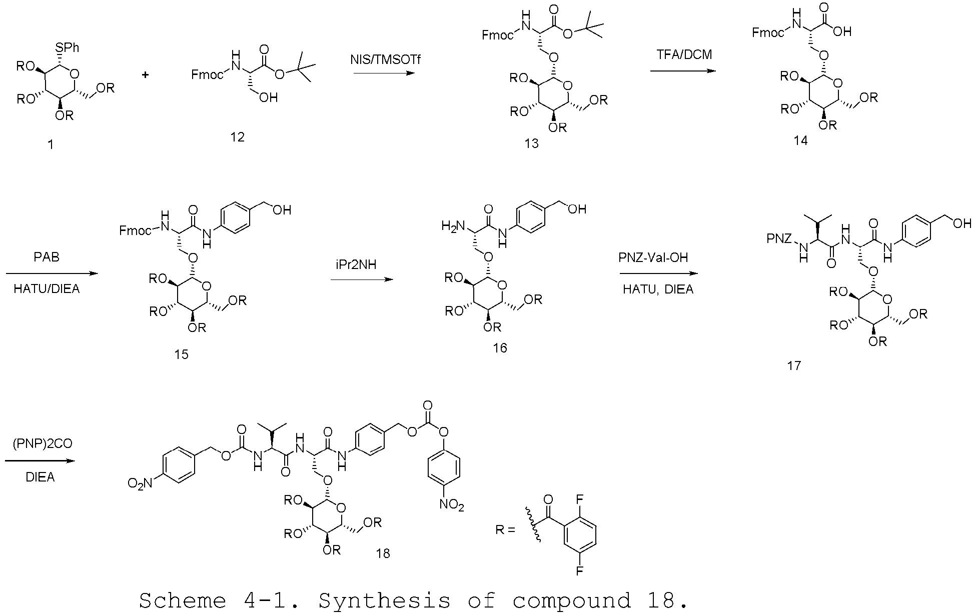

To a stirred solution of thioglycoside 1 (0.83 g) and compound 12 (0.38 g) in anhydrous DCM (10 mL) under argon atmosphere at 0 °C was added NIS (0.24 g), followed by TMSOTf (0.01 mL) and the solution was stirred at room temperature for 10 mins. The mixture was diluted with DCM (30 mL) and washed with Na₂S₂O₃ (10%, aq., 20 mL) and water (20 mL). The organic layer was concentrated under reduced and the residue was redissolved in TFA/DCM (1/1, v/v, 10 mL). After 30 min, the mixture was diluted with DCM (20 mL) and washed with water (10 mL x 4). The organic layer was evaporated to dryness to give the crude acid **14,** which was dissolved in DMF (10 mL). To this solution, p-aminobenzylalcohol (0.14 g) was added, followed by DIEA (0.35 mL) and HATU (0.38 g). The reaction was stirred at room temperature for 10 mins, and then diluted with EtOAc (30 mL). The mixture was washed with 0.5 M hydrochloric acid (15 mL) and water (20 mL). The organic layer was dried (over Na₂SO₄) and evaporated to dryness under reduced pressure. The residue was triturated with hexane/diethyl ether (1/1, 10 mL) to give the crude compound **15** as a tan solid, which was dissolved in DMF (5 mL). Diisopropylamine (5 mL) was added, and the mixture was stirred at room temperature for 2 h. The reaction was then concentrated under reduced pressure, and the residue was purified by RP-HPLC to give compound **16** (320 mg) as a white powder after lyophilization.

### Preparative HPLC method details:

Column: Phenomenex Gemini NX 5m, C18, 110 Å, 150 x 50 mm
Instrument: Shimadzu LC with CTC IFC
Mobile phase: A) water (0.1% TFA), B) acetonitrile
Gradient: 5-55% B over 20 min, flow 50 mL/min

The desired compound eluted at 19 min

To a solution of compound **16** (104 mg, 100 mmol) in DMF (2 mL), PNZ-Val-OH (30 mg) and DIEA (53 mL) were added, followed by HATU (40 mg). The mixture was stirred at room temperature for 10 min and purified directly by RP-HPLC to give compound **17** (104 mg) as a white powder after lyophilization.

### Preparative HPLC method details:

Column: Phenomenex Gemini NX 5m, C18, 110 Å, 100 x 30 mm
Instrument: Shimadzu LC with CTC IFC
Mobile phase: A) water (0.1% TFA), B) acetonitrile
Gradient: 10-70% B over 15 min, flow 50 mL/min

The desired compound eluted at 14 min

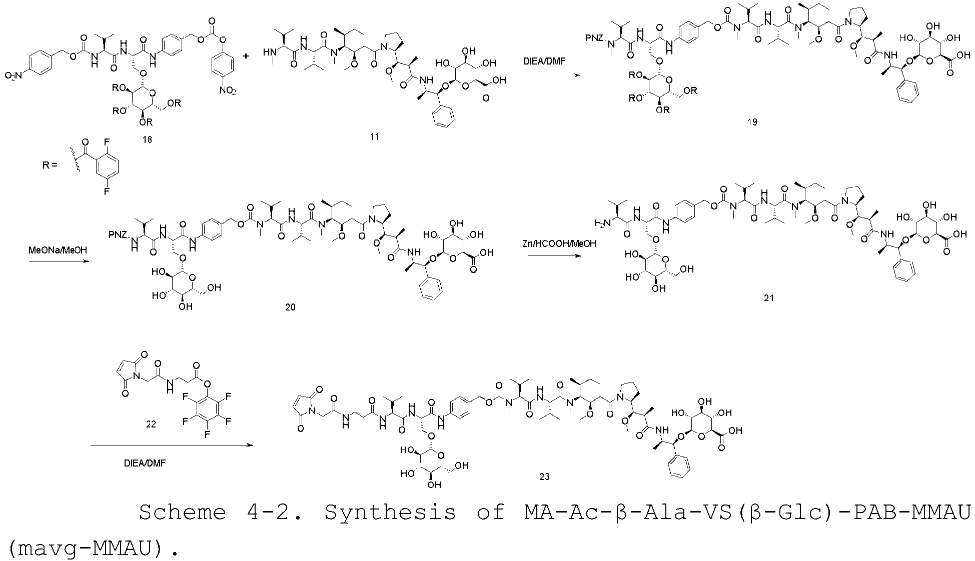

Compound **17** (62 mg) was dissolved in DMF (1 mL). To this solution, DIEA (9 µL) and bis PNP carbonate (20 mg) were added, and the mixture was stirred at room temperature for 24 h. Compound **11** (50 mg) in DMF (1 mL) was added, followed by DIEA (20 µL) and HOAt (3 mg). The mixture was stirred at room temperature for 48 h and the crude product was purified directly by RP-HPLC to give compound **19** (53 mg) as a white powder after lyophilization.

### Preparative HPLC method details:

Column: Phenomenex Gemini NX 5m, C18, 110 Å, 100 x 30 mm
Instrument: Shimadzu LC with CTC IFC
Mobile phase: A) water (0.1% TFA), B) acetonitrile
Gradient: 10-80% B over 15 min, flow 50 mL/min

The desired compound eluted at 15 min

To a solution of compound **19** (11 mg) in MeOH (1 mL) was added MeONa (25% in MeOH, 4 µL) and the mixture was stirred at room temperature for 20 min. LCMS analysis indicated that all the 2,5-difluorobenzoyl groups were removed. Formic acid (100 µL) was added, followed by Zn dust (30 mg) and the mixture was stirred at room temperature for 30 min. All the insoluble material was removed by centrifugation and the residue was purified directly by RP-HPLC to give compound **21** (6 mg) as a white powder after lyophilization.

### Preparative HPLC method details:

Column: Phenomenex Gemini NX 5m, C18, 110 Å, 100 x 30 mm
Instrument: Shimadzu LC with CTC IFC
Mobile phase: A) water (0.1% TFA), B) acetonitrile
Gradient: 5-40% B over 15 min, flow 50 mL/min

The desired compound eluted at 14 min

Compound **21** (10 mg) is dissolved in DMF (1 mL), and compound **22** (3 mg) is added, followed by DIEA (5 µL). The mixture is stirred at room temperature for 1 h and purified directly by RP-HPLC to give compound **23** (mavg-MMAU, GLD-22) as a white solid after lyophilization.

### Preparative HPLC method details:

Column: Phenomenex Gemini NX 5m, C18, 110 Å, 100 x 30 mm
Instrument: Shimadzu LC with CTC IFC
Mobile phase: A) water (0.1% TFA), B) acetonitrile
Gradient: 5-50% B over 15 min, flow 50 mL/min

### EXAMPLE 5 - Synthesis of mavu-MMAU

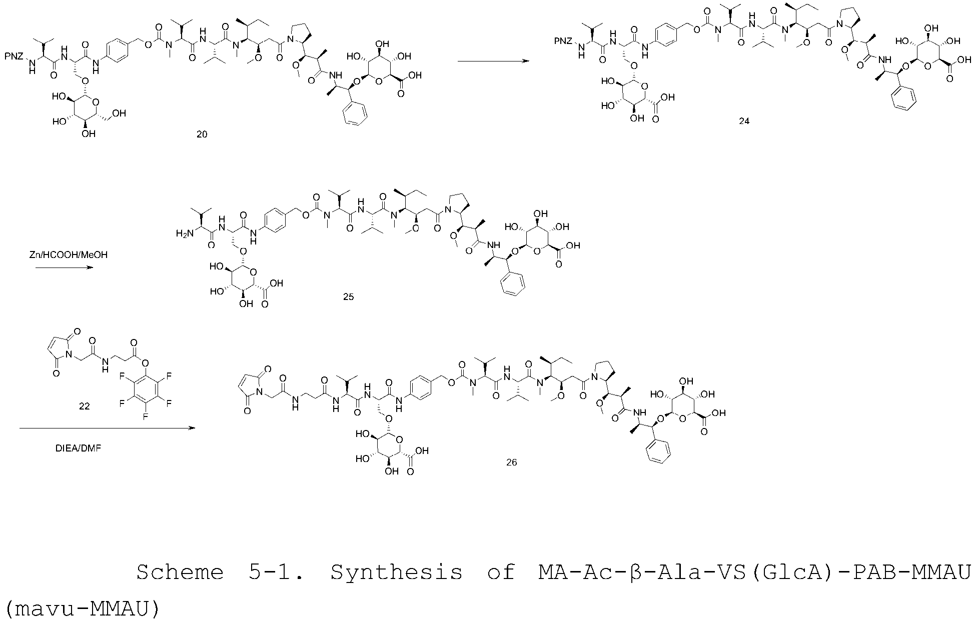

To a solution of compound **19** (22 mg) in MeOH (1 mL) was added MeONa (25% in MeOH, 7 µL) and the mixture was stirred at room temperature for 20 min. LCMS analysis indicated that all the 2,5-difluorobenzoyl groups were removed. Hydrochloric acid (1N, 40 µL) was added and the reaction mixture was concentrated to dryness under reduced pressure. The residue was dissolved in acetonitrile/water (6/4, v/v, 1 mL) and 40 µL of 0.67 M phosphate buffer (prepared by mixing Na₂HPO₄ and NaH₂PO₄ at 1:1 ratio) was added, followed by TEMPO (7 µL of 0.1 M solution in acetonitrile), bleach (Costco, 7%, 10 µL), and NaClO₂ (1 M in water, 20 µL). The reaction mixture was stirred at 35 °C for 24 h. More bleach (10 µL) and NaClO₂ (1 M in water, 20 µL) were added, and the reaction was continued to stir at 35 °C for 16 h. The mixture was purified by RP-HPLC to give compound **24** as a white powder (12 mg) after lyophilization.

### Preparative HPLC method details:

Column: Phenomenex Gemini NX 5m, C18, 110 Å, 100 x 30 mm
Instrument: Shimadzu LC with CTC IFC
Mobile phase: A) water (0.1% TFA), B) acetonitrile
Gradient: 5-60% B over 15 min, flow 50 mL/min

The desired compound eluted at 14 min

Compound **24** (12 mg) was dissolved in MeOH (1 mL) and Formic acid (100 µL) was added, followed by Zn dust (30 mg) and the mixture was stirred at room temperature for 30 min. All the insoluble material was removed by centrifugation and the residue was purified directly by RP-HPLC to give compound **25** (10 mg) as a white powder after lyophilization.

### Preparative HPLC method details:

Column: Phenomenex Gemini NX 5m, C18, 110 Å, 100 x 30 mm
Instrument: Shimadzu LC with CTC IFC
Mobile phase: A) water (0.1% TFA), B) acetonitrile
Gradient: 5-45% B over 15 min, flow 50 mL/min

The desired compound eluted at 14 min

Compound **25** (10 mg) was dissolved in DMF (1 mL), and compound **22** (3 mg) was added, followed by DIEA (5 µL). The mixture was stirred at room temperature for 1 h and purified directly by RP-HPLC to give mavu-MMAU **(26)** as a white solid (8 mg) after lyophilization.

### Preparative HPLC method details:

Column: Phenomenex Gemini NX 5m, C18, 110 Å, 100 x 30 mm
Instrument: Shimadzu LC with CTC IFC
Mobile phase: A) water (0.1% TFA), B) acetonitrile
Gradient: 5-50% B over 15 min, flow 50 mL/min

The desired compound eluted at 15 min

### EXAMPLE 6 - Characterization of synthesized MMAU and mavg-MMAU

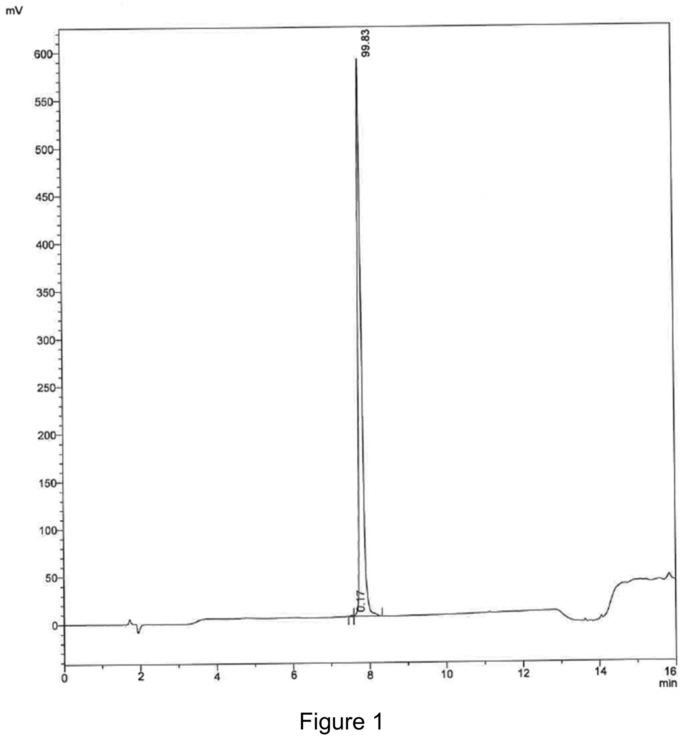

Figure 1 shows the RP-HPLC analysis of synthesized MMAU, indicating 99.83% purity. The analytical HPLC method details were:
Column: Phenomenex Gemini NX 5 µm, C18, 150 x 4.6 mm
Gradient: 10-60% acetonitrile over 16 min, UV detection at 220 nm

The desired compound eluted at 8 min

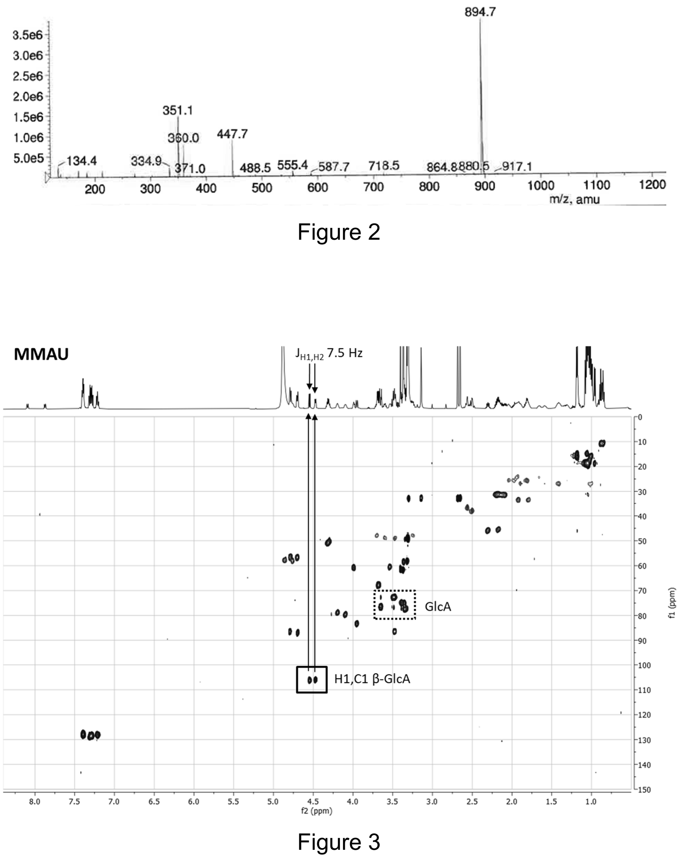

Figure 2 shows the ESI-MS analysis with the correct observed m/z 894.7 (calculated m/z 894.54 for the [M+H]⁺ ion).

The RP-HPLC analysis of synthesized mavg-MMAU indicated 99.76% purity. The analytical HPLC method details were:
Column: Phenomenex Gemini NX 5 µm, C18, 150 x 4.6 mm
Gradient: 20-70% acetonitrile over 15 min, UV detection at 220 nm

The synthesized mavg-MMAU was subjected to ESI-MS analysis with the correct observed m/z 1600.2 (calculated m/z 1599.8 for the [M+H]⁺ ion).

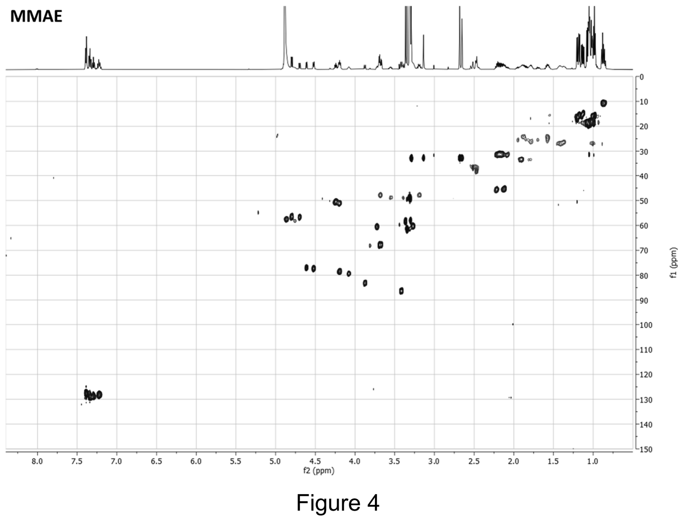

The synthesized MMAU was further characterized by ¹H and HSQC NMR spectroscopy measured at 22°C in CD₃OD at 600 MHz. The ¹³C-edited HSQC spectrum of MMAU (Figure 3) shows the expected signals from the β-glucuronic acid (boxed signals with solid line at about 4.5 ppm for the anomeric signals and dashed line for other signals) and no α-glucuronic acid signals were detected. The overall MMAU spectrum corresponds to reference MMAE data (Figure 4).Taken together, the characterization data shows chemical and stereochemical purity as well as correct identity for both MMAU and mavg-MMAU.

While there have been shown and described and pointed out fundamental novel features as applied to preferred embodiments thereof, it will be understood that various omissions and substitutions and changes in the form and details of the devices and methods described may be made by those skilled in the art without departing from the spirit of the disclosure. For example, it is expressly intended that all combinations of those elements and/or method steps which perform substantially the same function in substantially the same way to achieve the same results are within the scope of the disclosure. Moreover, it should be recognized that structures and/or elements and/or method steps shown and/or described in connection with any disclosed form or embodiments may be incorporated in any other disclosed or described or suggested form or embodiment as a general matter of design choice.

The applicant hereby discloses in isolation each individual feature described herein and any combination of two or more such features, to the extent that such features or combinations are capable of being carried out based on the present specification as a whole, in the light of the common general knowledge of a person skilled in the art, irrespective of whether such features or combinations of features solve any problems disclosed herein, and without limitation to the scope of the claims. The applicant indicates that the disclosed aspects/embodiments may consist of any such individual feature or combination of features. In view of the foregoing description it will be evident to a person skilled in the art that various modifications may be made within the scope of the disclosure.

It is obvious to a person skilled in the art that with the advancement of technology, the basic idea may be implemented in various ways. The embodiments are thus not limited to the examples described above; instead they may vary within the scope of the claims.

The embodiments described hereinbefore may be used in any combination with each other. Several of the embodiments may be combined together to form a further embodiment. A method, a product, an arrangement, or a use, disclosed herein, may comprise at least one of the embodiments described hereinbefore. It will be understood that the benefits and advantages described above may relate to one embodiment or may relate to several embodiments. The embodiments are not limited to those that solve any or all of the stated problems or those that have any or all of the stated benefits and advantages. It will further be understood that reference to 'an' item refers to one or more of those items. The term "comprising" or "including" is used in this specification to mean including the feature(s) or act(s) followed thereafter, without excluding the presence of one or more additional features or acts.

## Claims

1. A method for preparing a uronyl compound, wherein the method comprises
providing a compound represented by Formula I 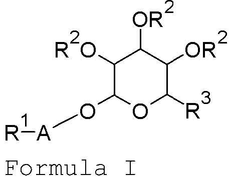
wherein R¹ is either H or a protecting group;
A is selected from A¹, A², A³, A⁴, and A⁵ 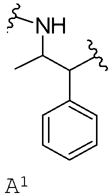 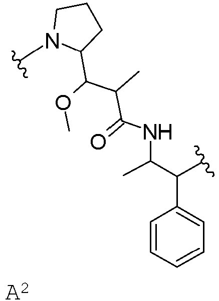 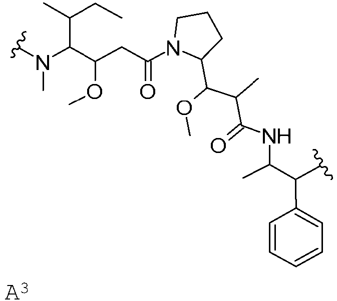 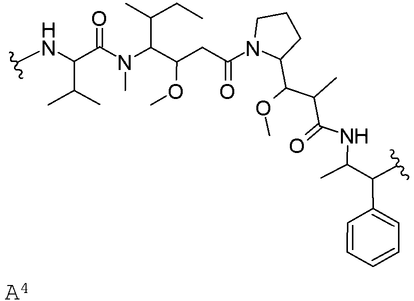 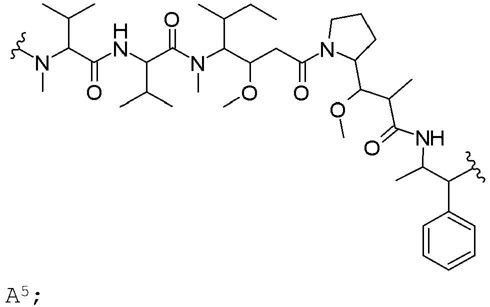
R³ is CH₂OH or CH₂OR²; and
each R² is independently selected from either H or a protecting group;
when R³ is CH₂OR², wherein R² is a protecting group, optionally removing the protecting group from R³; and
oxidizing the compound represented by Formula I in the presence of an oxidizing agent, a catalyst, water, and a bromide, such that R³ in the compound represented by Formula I is oxidized into COOH, wherein the oxidizing agent is sodium hypochlorite and the catalyst is 2,2,6,6-tetramethylpiperidine 1-oxyl (TEMPO).

2. The method according to claim 1, wherein the compound represented by Formula I is selected from the following: 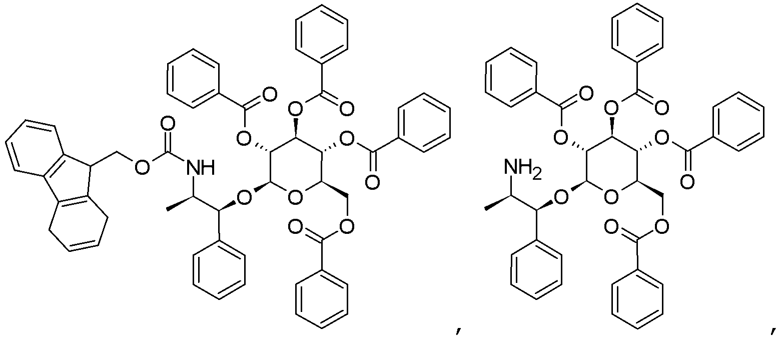 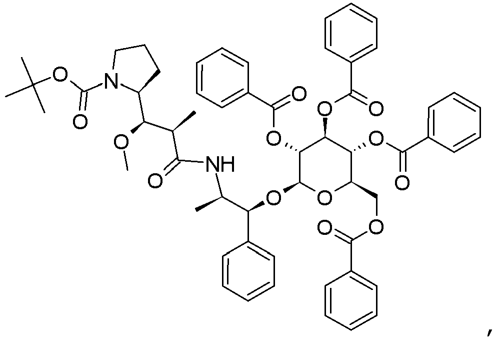 or

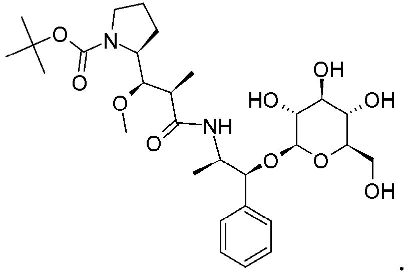

3. A method for preparing a uronyl compound, wherein the method comprises
providing a compound represented by Formula I 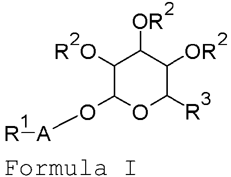
wherein R¹ is either H or a protecting group;
A is selected from A¹, A², A³, A⁴, and A⁵ 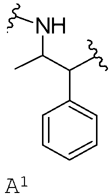 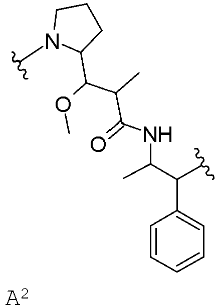 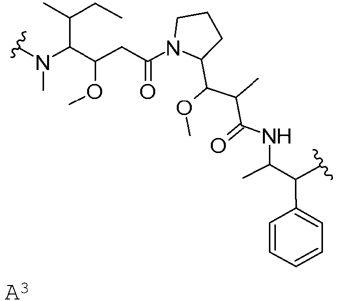 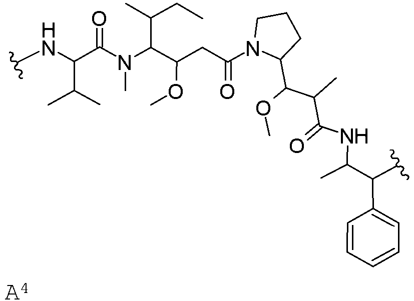 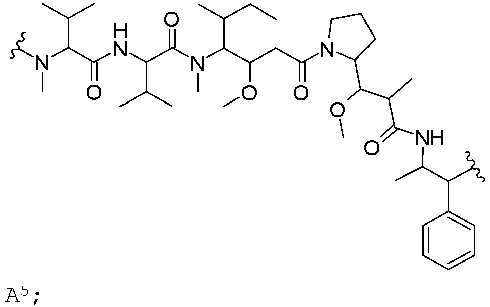
R³ is CH₂OH or CH₂OR²; and
each R² is independently selected from either H or a protecting group;
when R³ is CH₂OR², wherein R² is a protecting group, optionally removing the protecting group from R³; and
oxidizing the compound represented by Formula I in the presence of an oxidizing agent, such that R³ in the compound represented by Formula I is oxidized into COOH.

4. The method according to claim 3, wherein the compound represented by Formula I is oxidized in the presence of the oxidizing agent and optionally a catalyst; wherein the oxidizing agent is selected from the group of hydrogen peroxide, sodium hypochlorite, calcium hypochlorite, ozone, nitric acid, a permanganate compound, a halogen, a metal-catalyzed oxidation agent, gold nanoparticles, peroxidase activity-mimicking nanoparticles, and a glucose-oxidizing compound; and wherein the catalyst is selected from the group consisting of 2,2,6,6-tetramethylpiperidine 1-oxyl (TEMPO), a derivative thereof, glucose oxidase, and a glucose-oxidizing enzyme.

5. The method according to claim 3 or 4, wherein the oxidizing agent is selected from the group consisting of sodium hypochlorite and calcium hypochlorite, and the catalyst is selected from the group consisting of 2,2,6,6-tetramethylpiperidine 1-oxyl (TEMPO), 4-hydroxy-TEMPO, TEMPO methacrylate, 4-oxo-TEMPO, 4-amino-TEMPO, 4-acetamido-TEMPO, 4-carboxy-TEMPO, 4-hydroxy-TEMPO benzoate, 4-(2-iodoacetamido)-TEMPO, 4-maleimido-TEMPO, 4-isothiocyanato-TEMPO, 4-(2-bromoacetamido)-TEMPO, 4-methoxy-TEMPO, 4-cyano-TEMPO, 4-amino-4-carboxy-TEMPO, 4-phosphonooxy-TEMPO hydrate, and 2,2,6,6-tetramethyl-4-(methylsulfonyloxy)-1-piperidinooxy.

6. The method according to any one of claims 3 - 5, wherein the catalyst is 2,2,6,6-tetramethylpiperidine 1-oxyl (TEMPO) or a derivative thereof, the oxidizing agent is NaClO, and the compound represented by Formula I is oxidized in the presence of the oxidizing agent, the catalyst, KBr, and water.

7. The method according to any one of claims 1 - 6, wherein the compound represented by Formula I is oxidized at a temperature in the range of about 0 - 40 °C, 10 - 30 °C, 20 - 30 °C, 15 - 25 °C, or 20 - 25 °C.

8. The method according to any one of claims 1 - 7, wherein the compound represented by Formula I is oxidized at a pH in the range of about 7 - 10, or 8 - 9.

9. The method according to any one of claims 1 - 8, wherein the compound represented by Formula I is a glucosyl compound represented by Formula II 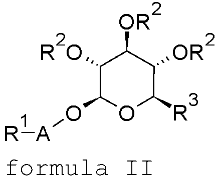 wherein R¹, R², R³, and A are as defined in claim 1 or 3.

10. The method according to any one of claims 1 - 9, wherein A is A¹ or A².

11. The method according to any one of claims 1 - 10, wherein the method further comprises adding one or more amino acid residues of the auristatin to the oxidized compound, thereby forming the auristatin moiety, such that a uronyl-monomethylauristatin compound is obtained.

12. The method according to claim 11, wherein the method further comprises
conjugating the uronyl-monomethylauristatin compound to a linker segment, thereby obtaining a linker-payload conjugate; and
conjugating the linker-payload conjugate to a targeting unit.

13. A compound represented by Formula I 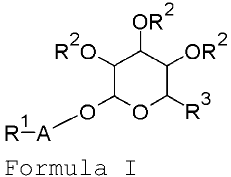
wherein R¹ is either H or a protecting group;
A is selected from A¹, A², A³, A⁴, and A⁵ 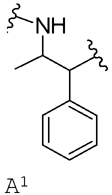 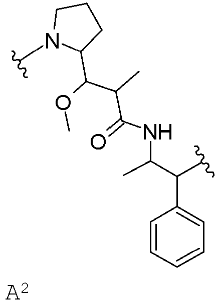 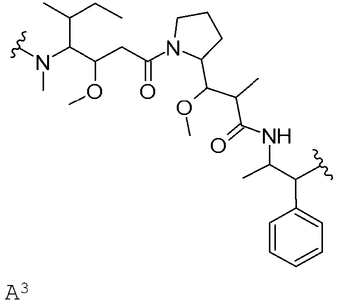 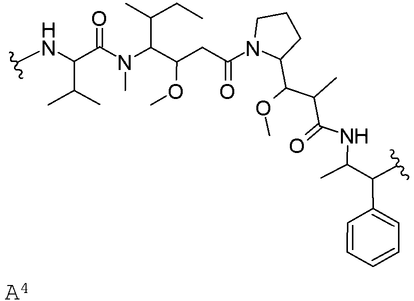 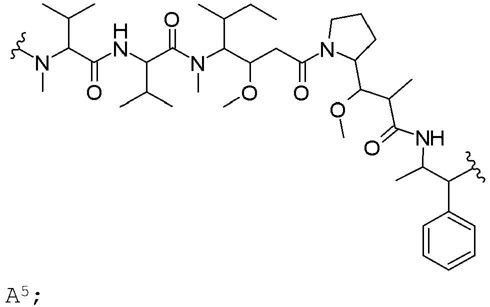
R³ is CH₂OH, CH₂OR², or COOH; and
each R² is independently selected from either H or a protecting group.

14. The compound according to claim 13, wherein A is A¹ or A².

15. The compound according to claim 13 or 14, wherein the compound is represented by any one of the formulas below 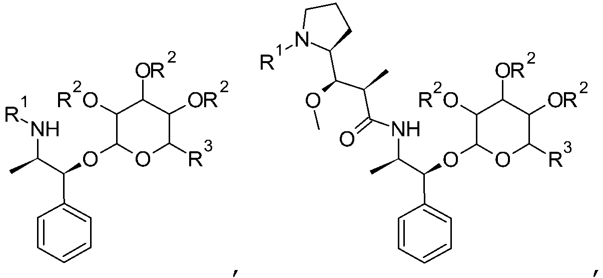 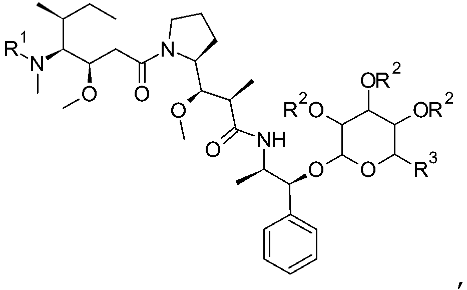 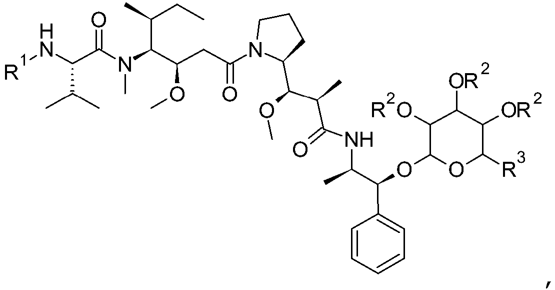 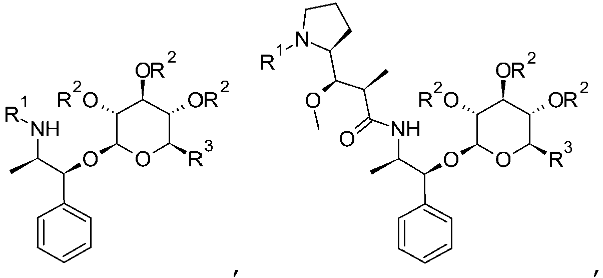 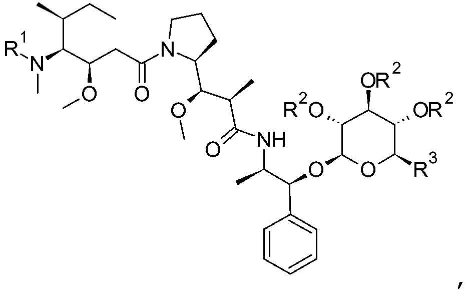 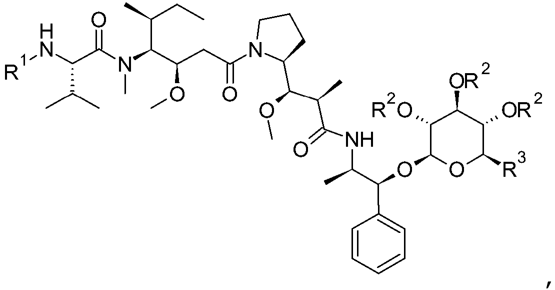 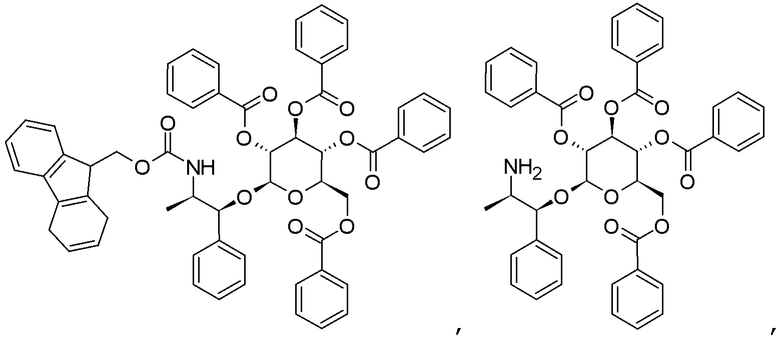 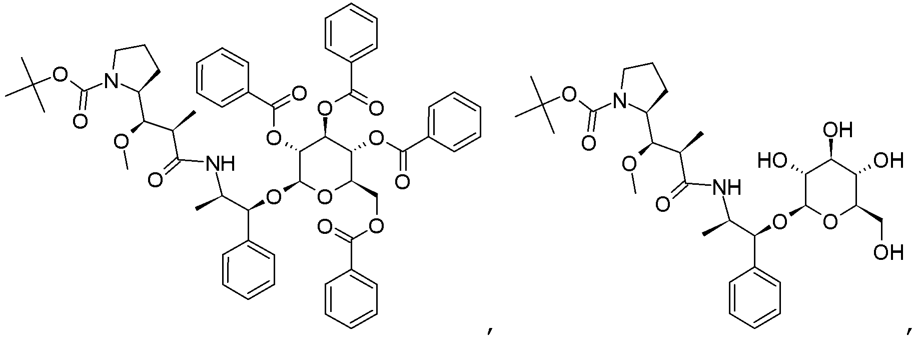 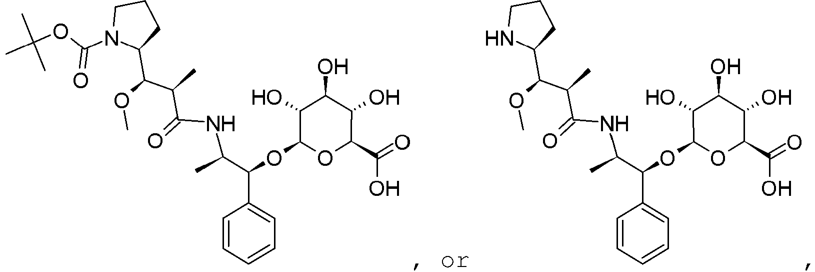 wherein R¹, R², and R³ are as defined in claim 13.
